(19) Europäisches Patentamt
European Patent Office
Office européen des brevets

(11) EP 3 288 893 B1

(12) # EUROPÄISCHE PATENTSCHRIFT

(45) Veröffentlichungstag und Bekanntmachung des Hinweises auf die Patenterteilung:
**27.02.2019 Patentblatt 2019/09**

(21) Anmeldenummer: **16722552.3**

(22) Anmeldetag: **26.04.2016**

(51) Int Cl.:
***C01B 21/24*** *(2006.01)* ***A61K 33/00*** *(2006.01)*
***A61Q 11/00*** *(2006.01)* ***A61Q 19/00*** *(2006.01)*
***A61K 8/19*** *(2006.01)*

(86) Internationale Anmeldenummer:
**PCT/EP2016/059312**

(87) Internationale Veröffentlichungsnummer:
**WO 2016/174043 (03.11.2016 Gazette 2016/44)**

(54) **MEHRSTUFIGES VERFAHREN ZUR NO-HERSTELLUNG**

MULTISTAGE METHOD FOR NO PRODUCTION

PROCÉDÉ DE PRODUCTION DE NO EN PLUSIEURS ÉTAPES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **29.04.2015 EP 15165747**

(43) Veröffentlichungstag der Anmeldung:
**07.03.2018 Patentblatt 2018/10**

(73) Patentinhaber: **BSN Medical Holding GmbH**
**20253 Hamburg (DE)**

(72) Erfinder:
- **HEMMRICH, Karsten**
**40667 Meerbusch (DE)**
- **ARSHI, Annahit**
**22527 Hamburg (DE)**
- **SCHULZE, Christian**
**21255 Tostedt (DE)**

(74) Vertreter: **FARAGO Patentanwalts- und Rechtsanwaltsgesellschaft mbH**
**Thierschstraße 11**
**80538 München (DE)**

(56) Entgegenhaltungen:
**EP-A1- 1 903 003**
**US-A1- 2013 022 691**
**US-A1- 2014 335 207**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 288 893 B1

## Beschreibung

### Gegenstand der Erfindung

Die vorliegende Erfindung betrifft ein Mehrschritt-Verfahren zur Herstellung von Stickstoffmonoxid (NO). Die Erfindung betrifft zudem das Verfahren in seiner Verwendung zur Behandlung von Erkrankungen, insbesondere von diabetisch bedingten Durchblutungsstörungen und Wunden der unteren Extremitäten.

### Hintergrund der Erfindung

Aus dem Stand der Technik sind zahlreiche Verfahren und Vorrichtungen zur Herstellung von NO bekannt.
Gemäß der EP 1 903 003 A1 kann NO durch Photolyse einer photolabilen NO-Vorstufe hergestellt werden, wobei die die Reaktion unter Anwesenheit von Radikalfängern und Antioxidantien zur Bildung von hochreinem NO verläuft. Bei diesem Verfahren ist in der Anwendung auf die NO-Erzeugung innerhalb von Flüssigkeiten in der Regel nur mit einem langsamen Anfluten der NO-Konzentration zu rechnen.
Gemäß der WO2013/063354 kann ein NO-freisetzendes Fußbad hergestellt werden, indem ein Polysiloxan-Polymer, das mit Diazeniumdiolatgruppen derivatisiert ist der Badlösung zugegeben wird. Dieses reagiert dann mit Wasser unter Bildung von NO. Da die NO-Generierung hierbei durch einen spontanen Zerfall der Polymerseitengruppen erfolgt, kann die Freisetzungskinetik hierbei nur unzureichend gesteuert werden. Zudem dauert es bei diesem Verfahren eine geraume Zeitspanne, bis ein therapeutisch relevanter NO-Spiegel aufgebaut ist.
Gemäß der US 2014/0335207 A1 wird ein NO-freisetzendes Medium hergestellt, indem ein Nitrit-haltiges Medium mit einem zweitem Medium gemischt wird, das mindestens zwei reduzierende Agenzien wie beispielsweise Milchsäure, Ascorbinsäure und Zitronensäure enthält. Durch die Zugabe eines Puffers soll die Produktion von NO verlängert werden, indem die Reaktion des Nitritsalzes mit dem reduzierenden Agenzien verlangsamt wird. Der Salzpuffer konkurriert dabei mit dem Nitritsalz um die Reaktion mit den reduzierenden Agenzien. Diese Mischung wird zur Behandlung mit NO auf die Haut von Säugetieren aufgetragen, wodurch es zur Aufnahme von NO über die Haut kommt.
Es besteht daher noch Bedarf an neuen Verfahren zur Herstellung NO-haltiger Lösungen, in denen schnell und dennoch in kontrollierter Weise NO in hoher Reinheit erzeugt werden kann.
Aufgabe der Erfindung ist es daher ein Verfahren zur NO-Herstellung bereitzustellen, die bezüglich mindestens einer der oben genannten Nachteile verbessert ist.

### Zusammenfassung der Erfindung

Diese Aufgabe wird gemäß der Erfindung dadurch gelöst, dass ein Verfahren zur Herstellung von Stickstoffmonoxid (NO) gemäß Anspruch 1 bereitgestellt wird. Spezifische Ausgestaltungen der Erfindung sind Gegenstand weiterer abhängiger Ansprüche.
Das erfindungsgemäße Verfahren vereinigt mehrere entscheidende Vorteile gegenüber den aus dem Stand der Technik bekannten Verfahren.
Es zeigte sich in überraschender Weise, dass dieses Verfahren den komplementären Anforderungen eine NO-Freisetzungskinetik gerecht wird. So kann im aziden Milieu sehr schnell eine therapeutisch relevante Konzentration an NO in dem Trägermedium aufgebaut werden, die dann nach pH-Werterhöhung über einen längeren Zeitraum in kontrollierter Weise aufrechterhalten werden kann.
Üblicherweise erschwert die kurze Halbwertszeit des NO den therapeutischen Einsatz. Mit dem erfindungsgemäßen Verfahren kann trotz der kurzen Halbwertszeit durch eine Stabilisierung des NOs in dem neutralen oder basischen Trägermedium der NO-Spiegel für eine ausreichende Zeitspanne aufrechterhalten werden.
Durch die Anwesenheit von Antioxidantien erlaubt das Verfahren die Herstellung von NO in einer Reinheit, wie sie für die therapeutische oder kosmetische Anwendung erforderlich ist.
Aus dem Stand der Technik sind zahlreiche pH-labile und photolabile NO-Donoren bekannt, wie beispielsweise Nitritsalze, NONOate oder Nitrosothiole, auf die der Fachmann hier zurückgreifen kann.
Aufgrund der hochkontrollierten Steuerung der Freisetzung kann das Verfahren in Vorrichtungen eingesetzt werden, die das NO nur in sehr geringen Mengen freisetzen. Dies ist insbesondere bei NO als hochpotentem bioaktivem Molekül ein entscheidender Vorteil. Zudem erlaubt dies die Entwicklung einer entsprechenden Vorrichtung (wie bspw. eine Wundauflage oder ein Fußbad) als Medizinprodukt (z.B. als sog. Medical device class III), insofern hier eine Vorrichtung vorliegt, bei der die Wirkung primär durch die mechanischen oder physikalischen Eigenschaften der Vorrichtung bedingt ist.
Durch einfache Anpassung des Verfahrens bezüglich NO-Donoren, Säuren und Bestrahlungsquellen kann es gezielt an die Behandlungserfordernisse angepasst werden.
Durch das erfindungsgemäße Verfahren kann auch auf eine externe Zuführung von NO verzichtet werden.

Bei der erfindungsgemäßen Verfahren handelt es sich um ein einfaches Verfahren mit größtenteils bekannten Substanzen, so dass es nicht nur kostengünstig sowie auf wenig komplexe Art und Weise durchzuführen ist, sondern auch bei geringer Fehleranfälligkeit einfach in der therapeutischen Anwendung ist.

Die mit dem Herstellungsverfahren betriebenen Vorrichtungen eröffnen im Hinblick auf die kennzeichnenden Parameter und auf die Materialauswahl weitere Freiheitsräume.

Zusammenfassend ermöglicht das erfindungsgemäße NO-Herstellungsverfahren NO-basierte Therapieformen, bei denen preiswert, zuverlässig, sicher und für den Anwender individualisierbar das hochreaktive, aber entsprechend instabile Gas NO in kontrollierter Weise applizierbar ist.

**Die Erfindung im Einzelnen**

Die Erfindung beinhaltet somit ein zweistufiges Verfahren, bei dem zunächst eine NO-Generierung im sauren Milieu induziert wird und nach einer ausgewählten Zeitdauer von 15 Sekunden bis 60 Minuten der pH-Wert anschließend um mindestens eine pH-Wertstufe erhöht wird, um die pH-abhängige NO-Neusynthese zu stoppen oder zu verringern und ein NO-haltiges Trägermedium bereitzustellen.

Durch die pH-Werterhöhung in den bevorzugterweise neutralen oder basischen Bereich unterbleibt die Neugenerierung von toxischen $NO_2$-Radikalen. Durch die erfindungsgemäße Anwesenheit des mindestens einen Antioxidans werden $NO_2$-Radikale und andere bei der NO-Generierung entstehenden Radikale eliminiert, so dass das Trägermedium mit hochreinem NO angereichert ist.

Ausgangspunkt des Verfahrens ist ein Trägermedium, das mindestens einen pH-labilen NO-Donor umfasst.

Weiterhin muss das Trägermedium zu dem Zeitpunkt, an dem ein azider pH-Wert die NO-Generierung erlaubt, zusätzlich mindestens ein Antioxidans umfassen. Hierzu kann das Antioxidans bereits im Schritt (a) im Trägermedium enthalten sein. Dies hat den Vorteil, dass die in dem Trägermedium enthaltenen Bestandteile auch schon während der Produktion und/oder Lagerung durch das anwesende, mindestens eine Antioxidans vor unerwünschter Oxidation geschützt sind. Dies kann gerade bei verfahrensgemäßen Vorrichtungen wie Wundauflagen oder Pflastern von Vorteil sein, da hier die Zugabe von weiteren Substanzen schwer möglich ist und diese eine ausreichende Lagerstabilität aufweisen müssen.

Alternativ kann das mindestens eine Antioxidans im Schritt (b) hinzugegeben werden. Dies ist vor allen dann sinnvoll, wenn es in nachteiliger Weise mit dem Trägermedium oder einem darin enthaltenen Bestandteil interagiert oder selber darin instabil ist. Weiterhin erlaubt dies die Möglichkeit, ein Antioxidans einzusetzen, das gleichzeitig als Säure die NO-Generierung induziert. Beispiele hierfür sind die Ascorbinsäure oder die Harnsäure.

<u>Trägermedium</u>

Als Trägermedium kann hier jedes Medium verwendet werden, dass in der Lage ist NO aufzunehmen und auch wieder abzugeben. Bevorzugt ist das Trägermedium ausgewählt aus der Gruppe, die Schaum, Gel, Creme und Flüssigkeit enthält. In bevorzugter Weise ist das Trägermedium eine Flüssigkeit und insbesondere eine wässrige Flüssigkeit.

Dieses Trägermedium kann selber als eigene Phase vorliegen, es kann aber auch in einem Substrat eingebettet sein. So kann eine wässrige Flüssigkeit als Badelösung vorliegen, sie kann aber auch im Rahmen einer Wundauflage in einer Schicht der Wundauflage enthalten sein, indem sie beispielsweise von einer Flüssigkeitsadsorbierenden Matrix aufgenommen worden ist.

<u>NO-Donor</u>

PH-labile NO-Vorstufen (NO-Donoren, NOD) sind im Stand der Technik bekannt und dem Fachmann geläufig.

In einer bevorzugten Ausführungsform der Erfindung sind die pH-labilen NO-Donoren ausgewählt aus der Gruppe enthaltend organische Nitrate, anorganische Nitrate, anorganische Nitrite, organische Nitritester wie Alkylnitrite, Schwefel-, Stickstoff- oder Sauerstoff-Nitrosoverbindungen, NO-Metall-Verbindungen und NO-chelatierende Substanzen.

Beispiele für pH-labile NOD umfassen anorganische Nitrite, Alkylnitrite wie Isopentylnitrit, Diazeniumdiolate (z.B. US Patente Nr. 7,105,502; 7,122,529; 6,673,338), trans[RuCl([15]aneN4)NO]$^{2+}$, Nitrosyl-Liganden, 6-Nitrobenzo[a]pyrol, S-Nitroso-Glutathion, S-Nitroso-Thiole, S-Nitroso-N-Acetyl-D-Penicillamin (SNAP), Nitroanilin-Derivate (siehe US 2013/0224083 A1), 2-Methyl-2-Nitrosopropan, Imidazoyl-Derivate, Nitratester, Hydroxylnitrosamin, Hydroxylamin, Hydroxyharnstoff oder Natrium-Nitroprussid.

In bevorzugter Weise ist der pH-labile NO-Donor ein anorganisches Nitritsalz, das zweckmäßigerweise eine pharmakologisch verträgliche Substanz darstellt. Als solche kommen beispielsweise Nitrite von Alkali- oder Erdalkalimetallen zur Anwendung. Beispielhaft sind hier genannt: $LiNO_2$, $NaNO_2$, $KNO_2$, $RbNO_2$, $CsNO_2$, $FrNO_2$, $Be(NO_2)_2$, $Mg(NO_2)_2$, $Ca(NO_2)_2$, $Sr(NO_2)_2$, $Ba(NO_2)_2$, oder $Ra(NO_2)_2$ und Kombinationen hiervon.

Besonders bevorzugt ist hierbei als NOD das $NaNO_2$, das in weiterhin bevorzugter Weise zusammen mit einer Kombination aus Ascorbinsäure und Trolox als Antioxidantien in dem Trägermedium eingesetzt wird.

Die Konzentration der Nitritsalze bezogen auf das Gesamtgewicht des sie enthaltenden Trägermediums kann hierbei bis zu 20 Gew.-% betragen, bevorzugt zwischen 0,25 und 10 Gew.-%, besonders bevorzugt zwischen 3 und 7,5 Gew.-%.

In einer alternativen Ausgestaltung kann auch ein Nitratsalz verwendet werden, bei denen eine enzymatische Umwandlung in das entsprechende Nitritsalz möglich ist. Bevorzugt sind hierbei Nitrate von Alkali- oder Erdalkalimetallen zur Anwendung. Beispielhaft sind hier genannt: $LiNO_3$, $NaNO_3$, $KNO_3$, $RbNO_3$, $CsNO_3$, $FrNO_3$, $Be(NO_2)_3$, $Mg(NO_2)_3$, $Ca(NO_2)_3$, $Sr(NO_2)_3$, $Ba(NO_2)_3$, oder $Ra(NO_2)_3$. Die Konzentration der Nitratsalze bezogen auf das Gesamtgewicht des sie enthaltenden Trägermediums kann hierbei bis zu 20 Gew.-% betragen, bevorzugt zwischen 0,25 und 10 Gew.-%, besonders bevorzugt zwischen 3 und 7,5 Gew.-%.

Antioxidans

Um die bei der NO-Generierung auftretenden mehrfach oxidierte Stickoxide, Sauerstoffradikalanionen oder Hydroxylradikale zu entfernen, ist es notwendig, dass das Trägermedium mindestens ein Antioxidans umfasst.

Nach Art des chemischen Wirkmechanismus werden Antioxidantien in Radikalfänger oder Reduktionsmittel unterschieden.

Bei Oxidationsreaktionen zwischen organischen Verbindungen treten vielfach kettenartige Radikalübertragungen auf. Hier werden Stoffe mit sterisch behinderten Phenolgruppen wirksam, die im Ablauf dieser Übertragungen reaktionsträge, stabile Radikale bilden, die nicht weiter reagieren, wodurch es zum Abbruch der Reaktionskaskade kommt (Radikalfänger). Zu ihnen zählen natürliche Stoffe wie die Tocopherole und synthetische wie Butylhydroxyanisol (BHA), Butylhydroxytoluol (BHT) und die Gallate. Sie sind insbesondere bei unpolaren Trägermedien anzuwenden.

Des Weiteren können auch Reduktionsmittel mit einem sehr niedrigen Standard-RedoxPotential von weniger als + 0,4 V (bei pH 7,0 und 25°C) eingesetzt werden. Typische Vertreter sind etwa Ascorbinsäure (-0,04 V bei pH 7 und 25°C), Salze der Schwefligen Säure (+0,12 V bei pH 7 und 25 °C) und bestimmte organische schwefelhaltige Verbindungen (z. B. Glutathion, Cystein, Thiomilchsäure), die vorwiegend in hydrophilen Trägermedien eingesetzt werden können.

In einer bevorzugten Ausführungsform ist das mindestens eine Antioxidans in der Lage das als NO-Donor im sauren Milieu vorliegende $HNO_2$ zu NO zu reduzieren. Hierzu muss das Antioxidans als Reduktionsmittel ein Standard-Redoxpotential von weniger als +1,0362 Volt, bevorzugt von weniger als + 0,5 Volt, besonders bevorzugt von weniger als + 0,2 Volt und insbesondere bevorzugt von weniger als 0 Volt aufweisen.

Das mindestens eine Antioxidans ist zweckmäßigerweise in der Lage das schädliche $NO_2$-Radikal zum $NO_2$-Anion zu reduzieren. Für eine effektive Eliminierung des $NO_2$ Radikals sollte das mindestens eine Antioxidans bevorzugt eine bimolekulare Reaktionskonstante k aufweisen, die größer als 1,0 x $10^6$ $M^{-1}s^{-1}$ und bevorzugt größer als 1,0 x $10^7$ $M^{-1}s^{-1}$ ist. Erfindungsgemäß geeignete Antioxidantien mit den dazugehörigen Reaktionskonstanten sind in Kirsch et al., 2002 (Biol. Chem 383; 389 -399, s. Tabelle 1) offenbart. Beispielhaft seien hier genannt: Captoprilthiolat, Kaffeesäure, Sinapinsäure, Ferulasäure, Lycopen, Zeaxanthin, Lutein, Astaxanthin, Canthaxanthin, Arachidonat, Gly-Tyr-Dipeptid, Tyrosin, Purine und Pyrimidine wie die Nucleobasen Adenin, Guanin, Cytosin, Thymin, Uracil und die entsprechenden Derivate und Analoga hiervon inklusive der sie enthaltenden Nucleoside und Nucleotide.

In einer weiteren Ausführungsform enthält das erfindungsgemäß verwendete Trägermedium, das in bevorzugterweise eine Flüssigkeit ist, neben dem Antioxidans auch einen Antioxidationssynergisten. Synergisten unterstützen die Wirkung von Antioxidantien, indem sie beispielsweise verbrauchte Antioxidantien wieder regenerieren (sog. "Redox cycling"). Durch Komplexierung von Metallspuren (Natrium-EDTA) oder Schaffung eines oxidationshemmenden pH-Wertes können Synergisten die antioxidative Wirkung eines Radikalfängers oder Reduktionsmittels verstärken. Typische Beispiele für Antioxidantionssysnergisten sind EDTA, 1-Hydroxyethan-1.1-diphosphonsäure, Citronensäure, Fumarsäure, Harnsäure und 2-(Hydroxymethyl)-1,4-benzyldiol.

Bei dem erfindungsgemäßen Herstellungsverfahren wird besonders bevorzugt das Ascorbat oder die Ascorbinsäure als Antioxidans eingesetzt.

Dem Fachmann sind zahlreiche Antioxidantien bekannt, welche in der Lage sind, mehrfach oxidierte Stickoxide, Sauerstoffradikalanionen, Hydroxylradikale oder aquatisierte Elektronen abzubauen oder zu neutralisieren. Diese wird er entsprechend der jeweiligen Zusammensetzung des Trägermediums auswählen.

Für apolare Trägermedien wie bspw. apolare Lösungsmittel, Cremes oder Gele eignen sich beispielsweise Antioxidantien wie Tocopherole, Tocotrienole, Tocomonoenole, Irganox®, Irgafos®, Butylhydroxyanisol (BHA) und Butylhydroxytoluol (BHT).

Für polare Trägermedien, wie bspw. wässrige Flüssigkeiten oder Hydrogele eignen sich wasserlösliche Vitamin E-Derivate wie Trolox oder alpha-AMG, organische schwefelhaltige Verbindungen wie Glutathion, Cystein, oder Thiomilchsäure oder auch organische Säuren wie Ascorbinsäure, alpha-Liponsäure, Hydroxyzimtsäuren wie p-Cumarsäure,

Ferulasäure, Sinapinsäure oder Kaffeesäure, oder Hydroxybenzoesäuren wie Gallussäure, Procatechusäure, Syringasäure oder Vanillinsäure.

Andere bevorzugte Antioxidantien umfassen polyphenolische Verbindungen wie Anthocyane, Flavonoide und Phytoöstrogene.

In einer bevorzugten Ausführungsform ist das mindestens eine Antioxidans aus Schritt (a) oder (b) ein Gemisch aus einem Vertreter der Reduktongruppe und einem Vertreter der 6-Hydroxy-Chromangruppe oder der Thiole. Es hat sich erfindungsgemäß herausgestellt, dass eine solche Antioxidantien-Kombination die bei der Reaktion entstehenden schädlichen Radikale besonders wirksam eliminieren kann, ohne dass die NO-Bildung hierdurch beeinträchtigt wird.

In einer bevorzugten Ausführungsform wird im Schritt (a) oder (b) eine Antioxidantien-Kombination gemäß der folgenden Tabelle eingesetzt. Die vorteilhafte Verwendung dieser Kombinationen beruht auf der Tatsache, das ein erstes Antioxidans bevorzugt das $HNO_2$ reduziert (Antioxidans I) und ein zweites Antioxidans bevorzugt das schädliche $NO_2$-Radikal abfängt (Antioxidans II). Die Tabelle stellt einerseits die allgemeine Stoffklasse vor, um dann beispielhaft einige bevorzugte konkrete Substanzkombinationen zu offenbaren.

| Antioxidans I | Antioxidans II |
|---|---|
| **Redukton** | **6-Hydroxychroman** |
| - Ascorbinsäure | - Trolox |
| - Isoascorbinsäure | - Trolox |
| - Erythroascorbinsäure | - Trolox |
| - Ascorbylstearat | - alpha-Tocopherol |
| - Ascorbylpalmitat | - alpha-Tocopherol |
| **Redukton** | **Thiol** |
| - Ascorbinsäure | - Cystein |
| - Isoascorbinsäure | - Cystein |
| - Erythroascorbinsäure | - Cystein |
| - Ascorbylstearat | - Cystein |
| - Ascorbylpalmitat | - Cystein |
| - Ascorbinsäure | - Glutathion |
| - Isoascorbinsäure | - Glutathion |
| - Erythroascorbinsäure | - Glutathion |
| - Ascorbylstearat | - Glutathion |
| - Ascorbylpalmitat | - Glutathion |

Erfindungsgemäß ist unter einem Vertreter der Reduktongruppe eine organisch-chemische Verbindung zu verstehen, die an den beiden Kohlenstoffatomen einer C=C-Doppelbindung zwei Hydroxylgruppen trägt ("Endiol") sowie zusätzlich direkt am benachbarten Kohlenstoffatom eine Carbonylgruppe aufweist. Die Doppelbindung dieser Endiole ist wegen der Konjugation mit der Carbonylgruppe stabilisiert; daher liegt im tautomeren Gleichgewicht ("Keto-Enol-Tautomerie") hauptsächlich die Endiolform und nicht die Ketoform vor. Als vinyloge Carbonsäuren reagieren Reduktone sauer. Die Reduktongruppe umfasst beispielweise Ascorbat und Derivate hiervon, Hydroxypropandial (Tartronaldehyd), trans-3,4-Dihydroxy-3-hexen-2,5-dion (DHHD) und 2,3-Dihydroxy-2-cyclopentenon (Reduktinsäure). In bevorzugter Weise wird als Vertreter der Reduktongruppe die Ascorbinsäure oder das Ascorbat und Derivate hiervon, wie Erythroascorbinsäure oder Ascorbylpalmitat eingesetzt.

Vertreter der 6-Hydroxychroman-Gruppe sind erfindungsgemäß Substanzen, die einen in 6-Position hydroxylierten Chromanring umfassen, der darüber hinaus an den anderen Positionen statt Wasserstoff einen oder mehrere weitere (bevorzugt Methyl)-Substituenten tragen kann. Typische Vertreter der 6-Hydroxychroman-Gruppe sind Tocopherole. Tocomonoenole und Tocotrienole und Derivate hiervon wie beispielsweise (RS)-6-Hydroxy-2,5,7,8-tetramethylchroman-2-carbonsäure (Trolox). In bevorzugter Weise wird als 6-Hydroxychroman-Vertreter das alpha-Tocopherol oder Trolox eingesetzt.

Thiole (auch Thioalkohole genannt) sind gemäß der Erfindung organisch-chemische Verbindungen, die eine

oder mehrere aliphatisch oder aromatisch gebundene Thiolgruppen (-SH) als funktionelle Gruppen tragen. Erfindungsgemäß sind Cystein und Glutathion als Thiole bevorzugt.

Die Endkonzentration der Thiole in dem Trägermedium liegt hierbei bevorzugt zwischen 1 und 1000 mM, besonders bevorzugt zwischen 20 und 200 mM und insbesondere bevorzugt zwischen 50 und 100 mM.

Für ein polares Trägermedium, wie bspw. ein wässrige Flüssigkeit oder ein Hydrogel, kann zweckmäßigerweise wasserlösliche Vertreter der vorabgenannten Gruppen kombinieren, also beispielsweise Ascorbat und (RS)-6-Hydroxy-2,5,7,8-tetramethylchroman-2-carbonsäure (Trolox), Ascorbat und Cystein, oder in bevorzugter Weise Ascorbat und N-Acetylcystein.

Für ein unpolares Trägermedium, wie bspw. eine Creme auf Öl oder Fettbasis, kann man zwei lipophile Vertreter der vorabgenannten Gruppen einsetzen, also beispielsweise Ascorbylpalmitat, Ascorbylstearat und alpha-Tocopherol einsetzen und bevorzugt eine Kombination aus Ascorbylpalmitat und alpha-Tocopherol oder Ascorbylstearat und alpha-Tocopherol.

Zweckmäßigerweise liegt das mindestens eine Antioxidans im Verhältnis zu dem NO-Donor in einem molaren Überschuss vor.

Bei einer Kombination aus zwei Antioxidantien mit $HNO_2$ und $NO_2$-Radikal Reaktionspräferenz (im Rahmen der Erfindung als Antioxidans I und Antioxidans II bezeichnet) ist es vorteilhaft, wenn sie eine einem molaren Verhältnis gemäß der folgenden Formel vorliegen:

$$\text{mol[NO-Donor]} < \text{mol [Antioxidans I]} < \text{mol[Antioxidans II]}.$$

Da die Eliminierung von $NO_2$ Radikalen eine besonders wichtige Aufgabe gerade im Bereich der therapeutischen und kosmetischen Anwendung darstellt, sollte das Antioxidans II aus Sicherheitserwägungen in einem größeren molaren Verhältnis vorliegen.

In bevorzugter Weise enthält das Trägermedium in Schritt (a) oder (b) die drei Komponenten: NO-Donor, Antioxidans I und Antioxidans II in einem molaren Verhältnis von 1 : 2-20 : 4-100, wobei das molare Verhältnis: Nitrit < Ascorbat < Trolox ist. Bevorzugt ist hier ein molares Verhältnis von 1 : 2-10 : 5-50, besonders bevorzugt von 1 : 3-8 : 5-20 und speziell ein Verhältnis von 1: 5 : 10.

In einer Ausführungsform der Erfindung enthält das Trägermedium und insbesondere in ihrer Ausgestaltung als wässrige Flüssigkeit zusätzlich einen oder mehrere der folgenden Stoffe: Katalysatoren, Detergentien, Puffersubstanzen, Chromophore, Substanzen, die das Prodrug stabilisieren wie bspw. Dimethylsulfoxid oder Ethanol, Substanzen, die die Halbwertszeit von NO erhöhen, wie bspw. in der US 2003/0039697 offenbart, NOD-Stabilisatoren, Antioxidantien, Farbstoffe, pH-Indikatoren, Pflegestoffe, Duftstoffe, pharmakologisch aktive Substanzen.

Der Fachmann wird in Hinblick auf den jeweiligen Verwendungszweck und basierend auf seinem allgemeinen Fachwissen geeignete Stoffe oder Stoffgemische auswählen. Hierbei wird er vor allem berücksichtigen, dass bei der Verwendung des Trägermediums zur topischen Anwendung physiologische verträgliche und/oder dermatologisch verträgliche Stoffe und Stoffgemische zur Anwendung kommen.

Säureaktivierung im Schritt (b)

Für die Spaltung des pH-labilen NO-Donors wird die Flüssigkeit auf einen sauren pH-Wert gebracht. Dieser pH-Wert liegt hierbei erfindungsgemäß so niedrig, dass er die Spaltung des pH-labilen NO-Donors unter Bildung von NO induziert. Der konkrete pH-Wert hängt von der pH-Labilität des NO-Donors und der erwünschten Zeitspanne für die NO-Generierung ab. Je geringer der pH-Wert, desto schneller wird im Trägermedium das NO erzeugt werden.

Gemäß der Erfindung beträgt der pH-Wert im Schritt (b) hierbei zwischen 0,0 und 6,9, bevorzugt zwischen 2,0 und 6,0, besonders bevorzugt zwischen 4,5 und 6,0 und insbesondere bei 5,0. Der optimale Wert für den pH-Wert ist wie oben bereits ausgeführt von dem jeweils verwendeten NO-Donor und der intendierten Reaktionsgeschwindigkeit abhängig und kann vom Fachmann entsprechend eingestellt werden.

In einer Ausführungsform der Erfindung wird bei dem Verfahren das für die NO-Freisetzung aus dem pH-labilen NO-Donor notwendige saure Milieu durch Zugabe einer Säure oder einen Puffers mit saurem pH-Wert (d.h. mit pH<7) erzeugt.

Als Säuren stehen dem Fachmann hierzu zahlreiche Säuren zur Verfügung. Dies umfasst sowohl Mineralsäuren wie HCl, $H_2SO_4$, $H_3PO_4$ oder $HNO_3$, als auch organische Säuren wie Essigsäure, Zitronensäure oder Milchsäure.

In einer besonderen Ausführungsform ist die Säure gleichzeitig ein Antioxidans, wie beispielsweise Ascorbinsäure oder Thiomilchsäure oder ein Antioxidationssynergist wie 1-Hydroxyethan-1.1-diphosphonsäure oder Harnsäure. Hierdurch kann auf die Anwesenheit eines Antioxidans im Schritt (a) verzichtet werden. Das Antioxidans wird als Säure im Schritt (b) hinzugegeben und kommt so gezielt ab dem Zeitpunkt zum Einsatz, ab dem durch die Spaltung des NO-Donors schädliche oder unerwünschte Radikale auftreten.

In einer weiteren Ausführungsform liegt die Säure in dem festen Trägermedium in fester Form vor und wird durch Zugabe von Wasser gelöst und damit deprotonierbar. Hierbei kann die Säure in Form von Pulver, Granulat, Nanopartikeln oder als an einem Polymer befindliche Säuregruppe vorliegen.

Photolatente Säure

In einer bevorzugten Ausführungsform der Erfindung wird die NO-Generierung im Schritt (b) durch Aktivierung eine photolatenten Säure initiiert, die durch Bestrahlung mit der elektromagnetischen Strahlung die Säure freisetzt, also zu einer Ansäuerung der Flüssigkeit führt. Dies hat den Vorteil, dass der Reaktion keine Säure von außen zugefügt werden muss, sondern die Ansäuerung durch einer in dem Trägermedium vorhandenen Substanz induziert werden kann.

Diese Ausführungsform ist insbesondere vorteilhaft, wenn im Schritt (e) weiteres NO durch einen photolytischen Prozess generiert wird, da dann schon die Lichtquelle bei dem erfindungsgemäßen Verfahren und der entsprechenden Vorrichtung vorgesehen ist.

Zudem ist es hier vorteilhaft, dass die Bestrahlung als initiales Ereignis eine länger anhaltende NO-Freisetzung induzieren kann und somit wie ein "Schalter" wirkt, der die erfindungsgemäße NO-Erzeugung startet.

Beispiele für photolatente Säuren sind z.B. Oniumsalze, wie Sulfonium- oder lodoniumsalze, sowie Oximsulfonsäureester. Solche Verbindungen sind in der Technik bekannt und in einer Vielzahl in der Literatur beschrieben.

Beispiele sind Triarylsulfonium- oder Diaryliodoniumsalze, z.B. unsubstituiert oder mit Alkyl- oder Alkoxysubstituenten mit den unterschiedlichsten Anionen, wie beispielsweise $HSO_4^-$, $PF_6^-$, SbF6$^-$, $AsF_6^-$, $Cl^-$, Br, $I^-$, $ClO_4^-$, $PO_4^-$, $SO_3CF_3^-$, Tosylat, oder einem Borat-Anion, wie etwa $BF_4^-$, oder $B(C_6F_5)_4^-$.

Oniumsalze sind z.B. von J.V. Crivello, K. Dietliker "Photoinitiators for Free Radical, Cationic & Anionic Photopolymerisation", Band III von "Chemistry & Technology of UV & EB Formulation for Coatings, Inks & Paints", 2. Ed., J. Wiley and Sons/SITA Technology (London), 1998 (insbesondere Seiten 464-466) beschrieben. Iodoniumsalze sind aus einer Vielzahl von Patentschriften bekannt, z.B. US 4151175, US 3862333, US 4694029, EP 562897, US 4399071, WO 98/46647 usw., und sind beispielsweise "symmetrische" oder "unsymmetrische" Diaryljodoniumverbindungen der Formel (C)

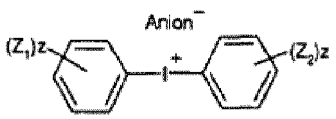
(C), worin

$Z_1$ und $Z_2$ gleich oder verschieden sind und beispielsweise lineares oder verzweigtes $C_1$-$C_{20}$-Alkyl, $C_1$-$C_{20}$-Alkoxy, Halogen, $C_2$-$C_{12}$-Alkenyl, Cycloalkyl darstellen; und z unabhängig voneinander für 0 bis 5, insbesondere für 0 oder 1, stehen, d.h. für den Fall, dass mehrere Reste $Z_1$ oder $Z_2$ vorliegen, z also grösser als 0 ist, alle $Z_1$ oder alle $Z_2$ nicht die gleiche Bedeutung haben müssen.

Weitere photolatente Säurespender sind von M. Shirai und M. Tsunooka in Prog. Polym. Sci., Vol. 21, 1-45 (1996), in Form einer Übersicht zusammengefasst.

Andere geeignete photolatente Säuren sind Oximsulfonate. Auch diese Verbindungen sind in der Technik bekannt und beispielsweise in US 5237059, EP 571330, EP 241423, EP 139609, EP 361907, EP 199672, EP 48615, EP 12158, EP 780729 offenbart.

Beispiele sind $\alpha$-(Methylsulfonyloxyimino)-4-methoxybenzylcyanid, $\alpha$-(Methylsulfonyloxyimino)-3-methoxybenzylcyanid, $\alpha$-(Methylsulfonyloxyimino)-3,4-dimethylbenzylcyanid, $\alpha$-(Methylsulfonyloxyimino)-thiophen-3-acetonitril, $\alpha$-(Isopropylsulfonyloxyimino)-thiophen-2-acetonitril, cis/trans-$\alpha$-(Dodecylsulfonyloxy-imino)-thiophen-2-acetonitril, ESACURE (Lamberti), IRGACURE (Ciba) e.g. IRGACURE® PAG103 (2-Methyl-$\alpha$-[2-[[[(n-propyl)sulfonyl]oxy]imino]-3(2H)-thienyliden]-benzylaceto-nitril, 2(5H)-Thienyliden]-Benzylacetonitril), IRGACURE® PAG108 (2-Methyl-$\alpha$-[2-[[[(n-octyl)sulfonyl]oxy]imino]-3(2H)-thienyliden]-benzyl-acetonitril), IRGACURE® PAG121 (2-Methyl-$\alpha$-[2-[[[(4-Methylphenyl)sulfonyl] oxy]imino]-3(2H)-thienyliden]-benzylacetonitril), IRGACURE® PAG203, Ethanon, 1,1'-[1,3-propandiyl-bis(oxy-4,1-phenylen)] bis-[2,2,2-trifluoro-bis[O-(propylsulfonyl) oxim], UVI (DOW Chemicals), CYRACURE (DOW Chemicals), und 2-(-Methoxystyryl)-4,6-bis(trichloro-methyl)-1,3,5-triazin (Sigma Aldrich).

Geeignet sind z.B. auch die in WO 2000/1097 A2 oder GB 2348644 beschriebenen Oximsulfonate. Oximverbindungen, welche andere Säuren als Sulfonsäuren abgeben sind ebenfalls geeignet und beispielsweise in WO 00/26219 offenbart.

Die vorstehende Aufzählung ist im Zusammenhang der vorliegenden Erfindung lediglich als beispielhaft und keineswegs als limitierend zu verstehen.

Erfindungsgemäß sind hier photolatente Lewissäuren bevorzugt. Bei der photolatenten Lewissäure handelt es sich um eine photochemisch aktive Substanz, das heißt, um eine Substanz, die in der Lage ist, aus eingestrahltem Licht

derart Energie aufzunehmen, dass diese Substanz infolge der Energieaufnahme in einer chemischen Reaktion verändert wird und dabei eine freie Lewissäure freisetzt.

Dazu weist die photolatente Lewissäure bei den Wellenlängen des einstrahlenden Lichts, deren Dosis jeweils zu überwachen ist, eine von Null verschiedene Absorption auf, so dass die Strahlung vollständig oder zumindest teilweise von der photolatenten Lewissäure absorbiert wird und diese in einen energetisch angeregten Zustand überführt. Der energetisch angeregte Zustand hat die Freisetzung der Lewissäure zur Folge. Hierdurch wird die Konzentration an freier Lewissäure in dem Trägermedium lokal erhöht, was in einer säureinduzierten Spaltung des pH-labilen NO-Donors resultiert.

Als photolatente Lewissäure kommt grundsätzlich jede Substanz in Frage, die zumindest in einem Wellenlängenbereich der Strahlung eine von Null verschiedene Absorption aufweist und die darüber hinaus in der Lage ist, infolge der Absorption der Strahlung eine Lewissäure freizusetzen, das heißt in einer chemischen Reaktion zu erzeugen oder anderweitig als freie Verbindung zur Verfügung zu stellen, beispielsweise in einem Desorptionsschritt oder aus einem Lewisaddukt. Bei der Lewissäure kann es sich beispielsweise um einen von der photolatenten Lewissäure abgespaltenen Teil handeln.

Als Lewissäuren werden alle elektrophilen Elektronenpaarakzeptoren verstanden, also alle Substanzen, die Elektronenpaare anlagern können, beispielsweise Moleküle und Ionen mit unvollständiger Edelgaskonfiguration, also einer Elektronenlücke.

Insbesondere gelten als Lewissäuren im Sinne dieser Erfindung auch Brønstedsäuren (klassische Säuren; Protonensäuren), das heißt Substanzen, die Protonendonatoren sind oder diese enthalten, wobei hierunter auch Protonen selber zu verstehen sind.

Beispiele für erfindungsgemäß einsetzbare photolatente Lewissäuren sind etwa aus WO 02/101462 A1 und WO 2005/097876 A1 bekannt, auf die hier ausdrücklich Bezug genommen wird.

Als latente Lewissäuren kommen gemäß WO 2005/097876 A1 insbesondere solche in Frage, die auf einer Verbindung der allgemeinen Formel $R^1$-CH*$R^0$-($A^6$)$R^2R^3R^4R^5$-OH basieren. Dabei stellt $A^6$ ein aromatisches Ringsystem mit sechs Ringatomen dar, das optional ein Heteroatom oder mehrere Heteroatome und/oder weitere anellierte Ringe enthalten kann. $R^1$ wird gewählt aus der Gruppe umfassend Wasserstoff, Alkylgruppen (insbesondere $C_1$-$C_{20}$-Alkylgruppen), Alkenylgruppen (insbesondere $C_2$-$C_{20}$-Alkenylgruppen), Arylgruppen (insbesondere unsubstituierte sowie einfach, zweifach oder dreifach mit $C_1$-$C_4$-Alkylgruppen substituierte Phenylgruppen, oder $C_1$-$C_4$-Alkoxygruppen. $R^2$, $R^3$, $R^4$ sowie $R^5$ werden unabhängig voneinander gewählt aus der Gruppe umfassend Wasserstoff oder funktionale Substituenten. R° wird gewählt aus der Gruppe umfassend $C_1$-$C_6$-Alkylgruppen, oder Gruppen der allgemeinen Formel -$Z^1$-$Q^1$ oder -$Z^2$-$Q^2$. $Z^1$ stellt dabei eine Einfachbindung dar oder ein verbrückendes Schwefelatom (-S-) oder Sauerstoffatom (-O-) oder eine verbrückende sekundäre Amingruppe (-NH-). $Q^1$ stellt dabei ein heterocyclisches Ringsystem mit 5 bis 9 Ringatomen dar, dessen Ringatome Kohlenstoff (C), Schwefel (S), Sauerstoff (O) und Stickstoff (N) sein können, wobei das Ringsystem zumindest zwei, bevorzugt drei, besonders bevorzugt wenigstens vier Kohlenstoffatome enthält. Insbesondere repräsentiert $Q^1$ Morpholin, Pyridin (das gegebenenfalls einfach bis dreifach mit C1-C2-Alkylgruppen oder Hydroxylgruppen substituiert sein kann), Mercaptobenzoxazol oder Mercaptobenzthiazol. $Z^2$ steht für eine $C_1$-$C_4$-Alkylengruppe, die mit einer $C_1$-$C_4$-Alkylgruppe oder mit $Q^3$ substituiert sein kann. $Q^2$ und $Q^3$ stellen hierbei unabhängig voneinander Phenylgruppen dar, die gegebenenfalls einfach bis dreifach mit $C_1$-$C_4$-Alkylgruppen, Hydroxylgruppen, $C_5$-$C_8$-Cycloalkylgruppen und/oder einem heterocyclischen Ringsystem mit 5 bis 9 Ringatomen substituiert sein können, dessen Ringatome Kohlenstoff (C), Schwefel (S), Sauerstoff (O) und Stickstoff (N) sein können, wobei das Ringsystem zumindest zwei, bevorzugt drei, besonders bevorzugt wenigstens vier Kohlenstoffatome enthält. Darüber hinaus kann das Wasserstoffatom H*, das an dem Kohlenstoffatom in Bezug auf den Substituenten R" in alpha-Stellung gebunden ist, bei Einwirkung von elektromagnetischer Strahlung in einer photochemischen Reaktion als Proton abgespalten werden.

Spezifische Beispiele für photolatente Lewissäuren sind in WO 02/101462 A1 beschrieben, die, ohne sich durch diese Beispiele einzuschränken, ausnahmslos eingesetzt werden können.

Als photolatente Säuren können ebenfalls die in WO 2003/050912 beschriebenen phenolischen Antioxidantien eingesetzt werden. Typische Beispiele hierfür sind etwa Verbindungen aus der Gruppe der Hydroxyphenylbenzotriazole, der Hydroxyphenyltriazine oder der Hydroxybenzophenone, die allesamt eine Hydroxylgruppe aufweisen, die an einem Phenylring in Bezug auf die Bindung zwischen dem Phenylring und dem Molekülhauptgerüst in ortho-Stellung angeordnet sind.

Schritt (c), der die NO-Generierung beinhaltet, weist eine Zeitdauer von zwischen 15 Sekunden und 1 Stunde, bevorzugt von zwischen 1 und 30 Minuten, besonders bevorzugt von zwischen 5 und 20 Minuten und besonders bevorzugt von zwischen 5 und 10 Minuten auf.

Die dabei generierte Menge an NO liegt bei einem festen Trägermedium zwischen 10 und 1000 ppm, bevorzugt zwischen 100 und 750 ppm und besonders bevorzugt zwischen 200 und 500 ppm.

Bei flüssigen Trägermedien ist die Konzentration des gebildeten NO bevorzugt aus molare Konzentration auszudrücken und liegt hierbei zwischen 0,01 und 2 mM, bevorzugt zwischen 0,05 bis 1 mM und besonders bevorzugt zwischen 0,1

und 0,5 mM.

pH-Werterhöhung

In einem der primären NO-Generierung gemäß Schritt (c) nachgeschaltetem Verfahrensschritt (d) wird der pH-Wert der Flüssigkeit um mindestens eine pH-Wertstufe erhöht. Diese pH-Werterhöhung kann erfindungsgemäß durch Zugabe einer Base, eines basischen Puffersystems oder durch Photoaktivierung einer photolatenten Base geschehen. Die erfindungsgemäße pH-Wert-Erhöhung weist weiterhin eine oder mehrere der folgenden Eigenschaften auf:

(i) eine Erhöhung des pH-Wertes auf pH 7,0 oder mehr;

(ii) eine Erhöhung des pH-Werts auf einen pH-Wert, der mit einer reduzierten NO-Generierung einhergeht, so dass die Menge an neugebildetem NO der Menge des im Trägermedium abnehmenden NOs entspricht.

Der pH-Wert wird erfindungsgemäß so stark erhöht, dass die Säure-induzierte NO-Generierung stark inhibiert wird oder sogar vollständig unterbleibt oder in einer besonderen Ausführungsform die NO-Generierung in einem reduziertem Maße derart erlaubt, dass diese Neubildung die Konzentrationsabnahme an NO (sei sie durch Zerfall, Abreaktion oder Freisetzung bedingt) kompensiert. In diesem Sinne sorgt die pH-Werterhöhung für die Aufrechterhaltung eines Fließgleichgewichts an NO. Zweckmäßigerweise führt die pH-Werterhöhung in Schritt (d) hierbei zu einem pH-Wert, der zwischen 4,0 und 12,0, bevorzugt zwischen 5,0 und 8,0, besonders bevorzugt zwischen 5,5 und 7,5 und insbesondere zwischen 6 und 7 liegt.

Als Basen stehen dem Fachmann hierzu zahlreiche Basen zur Verfügung. Dies umfasst sowohl anorganische Basen wie $NH_4OH$ als auch organische Basen wie aliphatische oder aromatische Amine.

In einer Ausführungsform wird zur pH-Werterhöhung eine Base verwendet wird, die ausgewählt ist aus der Gruppe enthaltend NaOH, KOH, $Ca(OH)_2$, $NH_4OH$ und Natriumhydrogencarbonat.

In einer alternativen Ausführungsform wird zur pH-Werterhöhung ein basischer Puffer verwendet, der ausgewählt ist aus der Gruppe enthaltend Phosphatpuffer, Barbital-Acetatpuffer, 4-(2-Hydroxyethyl)-1-piperazinethanesulfonsäure (HEPES)-Puffer, Tris(hydroxymethyl)-aminomethan(TRIS)-Puffer, 4-(2-Hydroxyethyl)-piperazin-1-propansulfonsäure (HEPPS)-Puffer, Barbital-Acetat-Puffer, Essigsäure-Acetat-Puffer, Kohlensäure-Silicat-Puffer, 2-(N-Morpholino)ethansulfonsäure (MES)-Puffer, Kohlensäure-Bicarbonat-Puffer, Citronensäure-Puffer oder Citrat-Puffer.

Photolatente Basen

In einer bevorzugten Ausführungsform wird für die pH-Werterhöhung eine photolatente Base verwendet, die durch Bestrahlung mit der elektromagnetischen Strahlung die Base freisetzt, also zu einer pH-Werterhöhung des Trägermediums (das bevorzugt eine Flüssigkeit ist) führt. Eine solche photolatente Base hat den Vorteil, dass auch hier keine Base extern dem System hinzugefügt werden muss, sondern dass auch hier die erfindungsgemäß optional eingesetzte (UV)-Lichtquelle den pH-Wert-Shift von außen auslösen kann.

Beispiele für photolatente Basen sind z.B. $\alpha$-Aminoacetophenone, Oniumsalze wie Sulfonium- oder Iodoniumsalze, sowie Oximsulfonsäureester. Solche Verbindungen sind in der Technik bekannt und in einer Vielzahl in der Literatur beschrieben.

Beispiele für erfindungsgemäß einsetzbare photolatente Basen sind etwa aus EP 0 898 202 A1, WO 94/28075 A1, WO 01/92362 A1, EP 0 970 085 A1 und WO 03/033500 A1 bekannt, auf die hier ausdrücklich Bezug genommen wird.

Geeignete photolatente Basen umfassen N-substitutierte 4-(o-Nitrophenyl) dihydropyridine, optional substitutiert mit Alkylether und/oder Alkylestergruppen, und quaternäre organische Bor-Photoinitiatoren. Beispiele für N-substitutierte 4-(o-Nitrophenyl)dihydropyridine sind N-Methyl-Nifedipin, N-Butyl-Nifedipin, N-butyl 2,6-dimethyl 4-(2-nitrophenyl)1,4-dihydropyridin 3,5-dicarboxylsäurediethylester und ein Nifedipin gemäß der folgenden Formel:

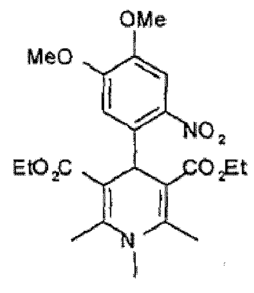

i.e., N-Methyl 2,6-Dimethyl 4-(4,5-Dimethoxy-2-Nitrophenyl)1,4-Dihydropyridin 3,5-dicarbonsäurediethylester. Beispiele für Organo-Bor-Verbindungen sind in der GB-A-2 307 473 offenbart, wie beispielsweise

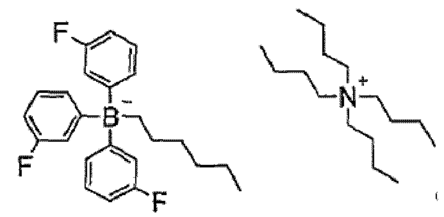

Aus dem Stand der Technik sind insbesondere die α-Amino-Acetophenon-Derivate als effiziente photolatente Basen bekannt. Beispiele für α-Amino-Acetophenone, die in dem erfindungsgemäßen Verfahren eingesetzt werden können, sind: 4-(Methylthiobenzoyl)-1-Methyl-1-Morpholinoethan (Irgacure®907ex, Ciba Spezialchemie) und (4-Morpholinobenzoyl)-1-benzyl-1-dimethylaminopropan (Irgacure®369ex, Ciba Spezialchemie), die auch in der EP 0 898 202 A1 offenbart werden. Bevorzugt ist ein α-Amino-Acetophenon der folgenden Formel:

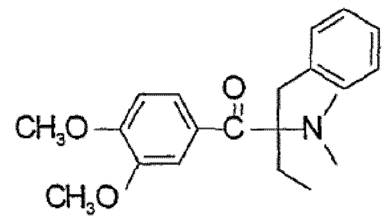

Die WO 94/28075 beschreibt UV-entblockbare Basen vom Amin, Ammonium oder Phosphan-Typ. Als blockierende Agenzien werden insbesondere alpha-Ketocarboxylsäuren, aromatische oder N-heterozyklische Ameisen-, Essig- oder Glyoxylsäurederivate, mit denen die Basen in ihre nicht reaktiven Salze umgewandelt werden und die durch Bestrahlung deblockiert werden können. Die WO 97/31033 beschreibt die photochemische Freisetzung von Basen mit einem $pK_a$ ~12, beispielhaft sei hier das N-Benzyloxycarbonyltetramethylguanidin erwähnt. Ionische Salze von α-Ammonium, α-Iminium oder α-Amidiniumketonen oder Alkenen, die bei Bestrahlung die korrespondierenden tertiären Aminbasen freisetzen, sind beispielsweise in der WO1998/38195 und der WO 2000/10964 offenbart. Die WO 1998/32756 offenbart α-Aminoketone, die bei Bestrahlung Amidinbasen freisetzen; korrespondierende α-Aminoalkene sind in der WO 1998/41524 offenbart.

Beispiele für geeignete Basen sind unter anderem tertiäre Amine und Amidine, wie Diazabicyclooctan, N-Aikylmorpholine, Tetramethylguanidin (TMG), Diazabicyclononen (DBN), Diazabicycloundecen (DBU) und Imidazol.

Besonders geeignete Amidine sind photolabile Diazabicyclononane, insbesondere 5-Benzyl-1,5-diazabicyclo[4.3.0]nonan, wobei der 5-Benzyl-Rest auch ein- oder mehrfach substituiert sein kann. Geeignete Substituenten am 5-Benzyl-Rest sind beispielsweise Halogenreste, wie Chlor oder Brom, Alkylreste, wie Methyl, Ethyl, oder Propyl, Nitrilreste, Nitrogruppen, Alkoxygruppen, wie Methoxy oder Ethoxy oder an den 5-Benzylrest ankondensierte aromatische Reste, wobei zum Beispiel aus einem 5-(Benzyl)rest ein 5-(Naphth-2-ylmethyl)rest oder ein 5-(Anthracen-9-yl-methyl)rest ableitbar ist. Auch kann anstelle des 5-Benzylrests beispielsweise ein 5-(Anthrachinon-2-yl-methyl)rest treten. Neben den möglichen Substitutionen am 5-Benzyl-Rest kann auch der Diazacyclononanrest weiter substituiert sein, wie zum Beispiel in 5-Benzyl-2-methyl-1,5-diazabicyclo[4.3.0]nonan. Neben den photolabilen Diazabicyclononanen, besteht auch die Möglichkeit photolabile Diazabicycloundecane, wie beispielsweise 8-Benzyl-1,8-diazabicyclo[5.4.0]undecane und dessen Derivate einzusetzen. Der 8-Benzyl-Rest kann analog dem 5-Benzyl-Rest des 5-Benzyl-1,5-diazabicyclo[4.3.0]nonans weiter substituiert sein oder ersetzt werden. Auch hier besteht die Möglichkeit einer weiteren Substitution am Diazabicyclononan-Rest.

In einer besonderen Ausführungsform wirkt das Antioxidans Ascorbat auch als photolatente Base. Wie die Erfinder herausgefunden haben, führt die Bestrahlung einer Ascorbat-Lösung mit UV-Strahlung, insbesondere hierbei mit $UV_A$-Strahlung zu einer Erhöhung des pH-Wertes.

Es können auch photolatente Basen eingesetzt werden, die zwei abspaltbare Basen in einem Molekül enthalten. Ein Vertreter dieser Art ist beispielsweise das 1,4-Bis(1,5-Diazabicyclo[4.3.0]nonanylmethyl)benzol. Die Synthese der oben genannten photolatenten Basen ist unter anderem in der WO 03/033500 A1 beschrieben.

Pharmakologische aktive Substanzen

In einer Ausführungsform der Erfindung enthält das Trägermedium und insbesondere in ihrer Ausgestaltung

als Flüssigkeit, Gel oder Creme eine oder mehrere pharmakologisch aktive Substanzen. Diese können die pharmakologische Wirkung des NOs unterstützen oder unabhängig von dem NO in einer für die entsprechende Erkrankung therapeutisch relevanten Weise wirken.

In einer Ausführungsform der Erfindung enthält das Trägermedium und insbesondere in ihrer Ausgestaltung als Flüssigkeit, Gel oder Creme eine oder mehrere der folgenden pharmakologisch aktiven Substanzen: Entzündungshemmer wie bspw. nichtsteroidale Antirheumatika (NSAIDs) oder Corticoide, Immunsuppressiva, Antibiotika, Antikoagulantien, Antithrombotika, antivirale Agenzien, Antimykotika, Lokalanästhetika und Analgetika.

Optionale Zugabe eines weiteren Antioxidans

In einer bevorzugten Ausführungsform der Erfindung wird mit der pH-Werterhöhung in Schritt (d) oder in einem nachfolgenden Schritt (e) mindestens ein Antioxidans hinzugegeben.

In einer Ausführungsform entspricht dieses mindestens eine Antioxidans dem im Schritt (a) oder (b) bereitgestellten mindestens einen Antioxidans. Auf diese Weise kann das während der NO Generierung verbrauchte Antioxidans wieder durch neues Antioxidans ergänzt werden.

In bevorzugter Weise ist das im Schritt (d) oder (e) neu hinzugegebene, mindestens eine Antioxidans ein Antioxidans, dass das vorher zugegebene mindestens eine Antioxidans regenerieren kann. Es wirkt somit als Antioxidationssynergist. Klassischerweise wird das Antioxidans bei der Reduktion der entsprechenden Substanzen selber oxidiert. Für eine Regeneration des Antioxidans muss dieses daher durch ein stärkeres Reduktionsmittel in die reduzierte Form umgewandelt werden (sog. "Redox cycling"). Bei Kenntnis des zu reduzierenden ersten Antioxidans muss der Antioxidationssynergist ein demgegenüber negativeres Standard-Redoxpotential besitzen. Für das bevorzugt verwendete Ascorbat mit eine Redoxpotential von +0,35 Volt ist somit beispielsweise das Cystein mit einem Redoxpotential von - 0,2 Volt (Cystein-Cystin; 25°C, pH 7.0) zur Regeneration geeignet.

In einer bevorzugten Ausführungsform handelt es sich hierbei um ein Antioxidans aus der Stoffklasse der Thiole. Bevorzugte Beispiele hierfür sind: Cystein, Glutathion, N-Acetylcystein, Dimercaptobernsteinsäure, Dimercaptopropansulfonsäure, Ethanthiol (Ethylmercaptan), Dithiothreitol (DTT), Dithioerythritol (DTE), Captopril, Coenzym A, Penicillamin, 1-Propanthiol, 2-Propanthiol, Homocystein, Mesna, Methanthiol (Methylmercaptan) und Thiophenol.

In einer besonderen Ausführungsform liegt ist nach Zugabe eines Thiols als Antioxidans im Schritt (d) die beiden Komponenten NO-Donor, welcher bevorzugt Nitrit ist, und Thiol in einem molaren Verhältnis von 1 : 1-20 vor. Bevorzugt ist hier ein molares Verhältnis von 1 : 2-8, besonders bevorzugt von 1 : 3-7 und speziell ein Verhältnis von 1: 5.

Photolytische NO-Generierung

In einer weiteren Ausführungsform der Erfindung wird bei dem Verfahren nach Schritt (d) oder (e) das Trägermedium zur photolytischen Zersetzung des NO-Donors unter Bildung von NO mit Licht bestrahlt wird. Eine nachgeschaltete photolytische NO-Generierung hat den Vorteil, dass ausgehend von der bereits erzeugten, physiologisch relevanten Menge an NO, ein Abnahme im NO-Gehalt (kumulativ bedingt durch die Weiterreaktion/Zerfall des NOs und die Freisetzung aus dem Trägermedium) durch die photolytisch-induzierte Neugenerierung in eleganter Weise kompensiert werden kann, das es keiner weiteren Zugabe von Substanzen zum Trägermedium bedarf und das Ausmaß der NO-Generierung leicht über die Bestrahlungsdauer und/oder Bestrahlungsintensität steuerbar ist.

Lichtquelle

Erfindungsgemäß kann in dem Verfahren eine Licht-Strahlungsquelle verwendet werden.

Eine Licht-Strahlungsquelle im Sinne der Erfindung erzeugt dabei eine elektromagnetische Strahlung, die das Spektrum des sichtbaren Lichts, des Infrarotlichts und insbesondere die UV-Strahlung beinhaltet. Die UV-Strahlung umfasst hierbei sowohl die $UV_A$ als auch die $UV_B$-Strahlung.

Die Art der Bestrahlung von NO-generierenden Ausgangssubstraten ist dem auf dem vorliegenden Gebiet tätigen Fachmann an sich bekannt. Es kann jegliche elektromagnetische Strahlung verwendet werden, welche in der Lage ist, photolabile NO-Derivate unter Bildung von Stickstoffmonoxid zu zersetzen. Beispielsweise kann im Rahmen der vorliegenden Erfindung die Herstellung von Stickstoffmonoxid mittels photolytischer Spaltung unter Verwendung einer $UV_A$-Strahlung mit Wellenlängen von beispielsweise 320 bis 400 nm erfolgen. Es kann jedoch auch elektromagnetische Strahlung jeder anderen Wellenlänge verwendet werden, die allein oder unter Zuhilfenahme chemischer, physikalischer oder biologischer Verfahren eine photolytische Spaltung von NO-generierender NO-Vorstufen (NO-Derivate) induziert.

Die Herstellung von Stickstoffmonoxid kann auch in Trägermedien, und hier bevorzugt in wässrigen Flüssigkeiten erfolgen, die mit inerten Gasen gesättigt sind. In solchen mit inerten Gasen (Stickstoff ($N_2$), Helium ($H_2$), Argon, usw.) gesättigten Lösungen hat das darin gelöste NO eine wesentlich längere Lebensdauer und kann auch in höheren

Konzentrationen in Lösung verbleiben. Allgemein wird angenommen, dass die maximale Löslichkeit von NO in wässrigen Lösungen ca. 2 mM beträgt. In diesem Zusammenhang können als wässrige Lösungen auch Kulturmedien oder Infusionsmedien oder Infusionspuffer verstanden werden.

Bei einer Vorrichtung zur Durchführung des erfindungsgemäßen Verfahrens kann die elektromagnetische Strahlung von einer Lichtquelle emittiert werden, die außerhalb und/oder innerhalb der Vorrichtung angebracht sein kann. Wichtig ist, dass die Lichtdurchflutung des Trägermediums mitsamt den das Stickstoffmonoxid freisetzenden Reaktionssubstanzen im Sinne eines induzierten Stoffzerfalls bzw. einer Freisetzung von Stickstoffmonoxid maximal ist. Die Quelle der elektromagnetischen Strahlung kann dabei eine mit entsprechenden Fluorochromen beschichtete Glüh- oder Gasentladungslampe (niedrigdruck- oder hochdruckentladend), Licht-emittierende Diode (LED), organische Licht-emittierende Diode (OLED), LASER oder jede andere elektromagnetische Strahlungsquelle sein, die in der Lage ist, aus entsprechenden chemischen Vorstufen bzw. Substraten NO zu generieren.

Für eine optimale Spaltung der in dem Trägermedium vorhandenen photolabilen NO-Vorstufen kann die Lichtquelle elektromagnetische Strahlung mit Wellenlängen von 100 bis 2000 nm emittieren oder elektromagnetische Strahlung jeder anderen Wellenlänge emittieren, die allein oder mit Unterstützung chemischer, physikalischer oder biologischer Verfahren eine Spaltung von Stickstoffmonoxid-Vorstufen und dadurch eine Bildung von Stickstoffmonoxid induzieren kann.

Vorzugsweise sollte bei einer photolytischen Spaltung daher die Vorrichtung in dem Bestrahlungsbereich aus einem Material aufgebaut sein, das die Eigenschaften des für eine optimale Freisetzung von Stickstoffmonoxid notwendige Energie einer elektromagnetischen Strahlungsquelle nicht beeinflusst oder aufgrund seiner Eigenschaften, die für eine lichtinduzierte Freisetzung von Stickstoffmonoxid notwendigen Lichteigenschaften erst schafft oder optimiert oder im Falle der pHabhängigen NO-Generierung den pH-induzierten Nitritzerfall fördert und optimiert.

Das zur Bestrahlung des photolabilen NO-Donors eingesetzte Licht liegt in einem Wellenlängenbereich, der von dem jeweiligen NO-Donor abhängig ist. So werden Nitrite zur Photolyse mit UV-Licht in einem Wellenlängenbereich zwischen 320 und 400 nm, bevorzugt zwischen 340 und 380 nm und besonders bevorzugt bei 365 nm bestrahlt. Bei S-Nitroso-Verbindungen ist eine Bestrahlung im UVA-Bereich bevorzugt (d.h. bei Wellenlängen zwischen 315 und 380 nm) aber auch Licht mit einer Wellenlänge von bis zu 1000 nm kann hierzu einer signifikanten Zerfallsrate führen.

Beachtlicherweise ist die optimale Wellenlänge für die Photolyse stark von Metallkationen abhängig. Insbesondere in der Anwesenheit von Ionen von Übergangsmetallen, so z. B. $Cu^{2+}$, können wässrige Nitritlösungen bei wesentlich längeren Wellenlängen Licht absorbieren, als es bei "reinen" Nitritlösungen der Fall ist und somit das Nitrition auch durch Licht in Wellenlängen von 400 - 450 nm und noch anderen Wellenlängen $\geq$ 450 nm unter NO-Freisetzung gespalten werden. Auch bei S- und N-nitrosierten chemischen Verbindungen kann aufgrund der relativ schwachen Bindungsenergie zwischen NO und dem Restmolekül ebenfalls durch elektromagnetische Strahlung $\geq$400 nm diese Verbindungen unter NO-Freisetzung photolytisch gespaltet werden.

Vorrichtung

Zur Durchführung des erfindungsgemäßen Verfahrens eignen sich Vorrichtungen ausgewählt aus der Gruppe enthaltend Badevorrichtung, Pflaster, Wundauflage, Inhalator, Munddusche und Spray. Die Vorrichtung kann hierbei umfassen:

(a) Ein wässriges Puffersystem als Trägermedium umfassend ein Nitritsalz, Ascorbat und Trolox;(b) Ein davon getrenntes Kompartiment mit einer Säure;(c) Mittel zur kontrollierten Freisetzung des in der Vorrichtung erzeugten NO; und(d) optional ein Bestrahlungseinrichtung zur Photolyse von NO-Donoren

.

Therapeutische oder kosmetische Verwendung

In einem besonderen Aspekt wird eine Vorrichtung zur Durchführung des erfindungsgemäßen Verfahrens bereitgestellt, die zur Verwendung bei der Behandlung oder Prävention von Erkrankungen geeignet ist, wobei der Patient dem aus der Vorrichtung freigesetzten NO ausgesetzt wird.

Bevorzugterweise erfolgt diese Behandlung mittels äußerlicher oder topischer Anwendung. So kann durch Auflegen eines NO-freisetzenden Pflasters oder einer Wundauflage auf einem therapiebedürftigen Hautareal dieses Areal gezielt behandelt werden.

Alternativ kann bei einer Badevorrichtung der erkrankte Körperteil durch Eintauchen in die NO-haltige Flüssigkeit behandelt werden oder aber durch Besprühen, Begießen oder Übergießen mit der NO-haltigen Flüssigkeit.

Die Vorrichtung kann hierbei insbesondere zur Anregung des Stoffwechsels von Geweben durch äußerliche Anwendung, im Bereich der Dermatologie zur Behandlung von chirurgischen oder unfallbedingten Wunden, chronischen,

nicht- bzw. schlechtheilenden und/oder bakteriell bzw. Pilzbefallenen Wunden sowie zur Behandlung von dermatologischen Erkrankungen aus dem Formenkreis der entzündlichen, immunologisch gesteuerten bzw. Autoimmunerkrankungen verwendet werden.

Bevorzugt ist die mit der Vorrichtung behandelte Erkrankung ausgewählt aus der Gruppe enthaltend neuropathische Schmerzen, Krampfadern, Ischämien und thrombopathische Erkrankungen, Allergien, Hautinfektionen, Hautentzündungen, atopischen Dermatitis insbesondere Neurodermitis, Dermatomyositis und Pemphigus vulgaris; Wunddefekte, wie der chronischen diabetisch-neuropathischen Ulcus, Ulcus cruris, Dekubituswunden; primär heilende Wunden, sekundär heilende infizierte Wunden, Brandwunden, Hidradenitis supparativa (Akne inversa), Warzen, Windelausschlag, Rasurbrand, Komplikationen bei Hauttransplantationen, erektile Dysfunktion, Angina pectoris, Herzinsuffizienz, Linksherzinsuffizienz, Koronare Herzkrankheit, Pektanginöse Symptome nach Myokardinfarkt, Analfissur, Krämpfe der glatten Muskulatur des Oesophagus, Menstruationsbeschwerden, Reynaud-Syndrom, Buerger-Syndrom, periphere arterielle Erkrankung (PAD), periphere arterielle Verschlusskrankheit (pAVK), entzündliche und Autoimmunerkrankungen der Haut (Psoriasis, Dermatiden, Neurodermitis), Pilzerkrankungen der Haut, bakterielle, mikotische und parasitäre Erkrankungen der Haut (z.B. Leishmaniose), Tinea cruris und Tinea inguinalis.

Mit dem erfindungsgemäßen Verfahren hergestellte Lösungen können bevorzugt in Form eines Inhalationssprays zur Behandlung obstruktiver Lungenerkrankungen verwendet werden. Weiterhin können sie zur Induktion einer lokalen Vasodilatation von verengten oder verschlossenen Blutgefäßen eingesetzt werden. Hierbei ist es bevorzugt, die Lösung direkt in das Herz zu applizieren, beispielsweise durch eine endoskopische Maßnahme.

Mit der Vorrichtung können lokale Durchblutungserkrankungen beim Tier, wie beispielsweise die Hufrehe beim Pferd behandelt werden und dabei generell veterinärmedizinische Erkrankungen, die den hier aufgelisteten Humanerkrankungen entsprechen oder nahekommen.

Die Vorrichtung kann auch zur Behandlung einer Muskeldystrophie (MD) eingesetzt werden. Behandelbare MD-Formen umfassen hier: MD-Duchenne, MD-Becker-Kiener, Emery-Dreifuss_MD-Typ 1, Skapulopereonale MD, reducing body myopathy (RBM), Gliedergürteldystrophien, kongenitale Muskeldystrophien, distale Muskeldystrophien, "Vocal cord and pharyngeal weakness with distal myopathy" (VCPDM), Myofibrilläre Myopathien und Myotone Dystrophien.

Eine mit der Vorrichtung behandelbare Entzündung kann eine bakterielle, virale, mykotische oder parasitäre Infektion darstellen. Die bakterielle Infektion kann hierbei beispielsweise durch ein Bakterium verusacht werden, das ausgewählt ist aus der Gruppe enthaltend S. aureus, B. circulans, B. cereus, E. coli, P. vulgaris, P. acnes, S. pyogenes, S. enterica, V. anguillarum, K. pneumoniae, P. piscicida, P. aeruginosa, A. tumefaciens, M tuberculosis, and M ulcerans. Die Pilzinfektion kann durch einen Pilz hervorgerufen werden, der ausgewählt ist aus der Gruppe enthaltend T. equinum, C. Albicans, F. oxysporum, R. solani, B. cinerea, und A. jlavus. Bei der zu behandelnden Pilzinfektion kann die Haut oder Nagel gemäß einer Onychomycosis befallen sein. Virale Infektionen können durch eine der folgenden Virenfamilien hervorgerufen werden: Poxviridae, Rotaviren, Papillomaviren, Parvoviren, und Varicella-Viren. Bevorzugt kann die NOfreisetzende Vorrichtung zur Behandlung von Hautinfektionen eingesetzt werden, bei denen der Virus Molluscum contagiosum involviert ist. Die parasitäre Infektion kann beispielsweise durch einen Parasit der folgenden Gattungen entstehen: Plasmodium, Leishmania, Schistosoma, Austrobilharzia, Heterobilharzia, Ornithobilharzia oder Cryptosporidium. Hervorzuheben ist hier der Erreger Plasmodium falciparum.

Die Vorrichtung kann zur Behandlung der anfallsartigen bei der Sichelzellanämie auftretenden Durchblutungsstörungen (Sichelzellkrisen) verwendet werden. Für den in solchen Fällen eingesetzten Wirkstoff Hydroxyharnstoff wird vermutet, dass er bei den Erythrozyten die Ausbildung der deoxygenierten T-Variante hemmt, und so die Umwandlung in den Sichelzellphänotyp verhindert. Durch Anbindung des freigesetzten NOs an das Hämoglobin entsteht hingegen die nichtsichelzellbildende R-Variante was mit einer Verbesserung der Durchblutung und sogar einer Unterbindung der Sichelzellkrisen einhergehen kann.

Weiterhin kann die Vorrichtung zur Behandlung von Haarausfall und hierbei insbesondere der androgenetischen Alopezie eingesetzt werden. Die Behandlung schließt hier sowohl eine Verlangsamung oder einen Stopp des Haarausfalls mit ein und sogar das Neuwachstum von Haaren. Weitere Formen des Haarausfalls, die erfindungsgemäß behandelt werden können, umfassen Alopecia praematura, Alopecia areata, Alopecia areata atrophicans, Alopecia totalis, Alopecia universalis, diffuse Alopezie, Alopecia actinica, Alopecia mechanis wie Alopecia liminaris, Alopecia marginalis frontalis traumatica, Alopecia seborrhoica, Alopecia muciosa und Alopecia parvimaculata. Analog zur Wirkungsweise des Medikaments Minoxidil sollte das NO hierbei durch erhöhte Durchblutung der Kopfhaut eine verstärkte Versorgung der Haarfollikel mit Blut, Sauerstoff und Nährstoffen mit sich bringen.

Die Vorrichtung kann beispielsweise wie folgt angewendet werden:1.) auf offenen Wunden, da sich überraschenderweise herausgestellt hat, dass die erfindungsgemäße Anwendung nicht hautirritierend wirken;2.) für die MRSA Prophylaxe in Risikopatienten; oder3.) als synergistische Anwendung mit konventionellen Antibiotika, da sich überraschend herausgestellt hat, dass in Folge einer NO-Einwirkung, die konventionellen Antibiotika die verbleibende Entzündung effektiv zu bekämpfen vermag.

Bevorzugt wird die Vorrichtung zur Behandlung chronischer Wunden der unteren Extremitäten von Diabetikern

verwendet. Hierbei kann zudem durch die Behandlung im Sinne einer Prophylaxe das Entstehungsrisiko chronischer Wunden sowie die Anzahl medizinischer Amputationen verringert werden. Dadurch gehen die Reduktion der neuropathischen Beinschmerzen und die Herstellung eines verbesserten Wundmilieus mit einer spürbar verbesserten Lebensqualität der Patienten einher. Darüber hinaus ist durch eine Verkürzung der Wundversorgung eine signifikante Minderung der Behandlungskosten zu erwarten.

Zudem könnte es möglich sein, durch Behandlung größerer Körperareale auch systemische Erkrankungen, wie z. B. den erhöhten Blutdruck (Hypertonie) und verwandte hämodynamische Erkrankungen zu adressieren.

Die Vorrichtung kann ebenfalls zur Behandlung schlechtheilender Wunden eingesetzt. Eine gestörte arterielle Durchblutung und/oder venöse Rückflussstörungen sind maßgebliche Ursachen in der Entstehung sowie Chronizität von Wunden der unteren Extremitäten. Eine NO-bedingte arterielle Vasodilatation verbessert die Durchblutung des betroffenen Gewebes und durch die antithrombogene Wirkung des NO wird ein venöser Rückfluss des Blutes wesentlich gefördert bzw. erleichtert. Die NO-abhängige Verbesserung beider hämodynamischer Parameter stellt den entscheidenden therapierelevanten Aspekt einer lokalen sowie systemischen Wirkung dar, die das Risiko der Entstehung von Wunden signifikant mindert bzw. deren Heilung wesentlich beschleunigt. Das mittels der Vorrichtung dem zu behandelnden Körperteil zugeführte NO kann daher erfolgreich zur Therapie schlechtheilender Wunden angewendet werden.

Insbesondere wird die Vorrichtung zur Behandlung des diabetischen Schmerz der unteren Extremitäten, also von Fuß und/oder Bein, eingesetzt. Der diabetische Schmerz ist ein sehr häufiges Ereignis im Verlauf einer Diabeteserkrankung. Der diabetischer Fuß-/Beinschmerz ist ein Ergebnis langandauernder erhöhter Blutglukosekonzentrationen, die die Grundursache für die während einer Diabeteserkrankung beobachteten Nerven- sowie Gefäßschädigung ist. Eine NO-bedingte arterielle Vasodilatation verbessert die Durchblutung des betroffenen Gewebes und hilft die Schmerzweiterleitung im Sinne einer Schmerzminderung zu beeinflussen. Das mit der Vorrichtung von außen dem Fuß und/oder Bein zugeführte NO kann daher erfolgreich zur Therapie des diabetischen Fuß-/Beinschmerzes angewendet werden.

Die Vorrichtung wird speziell zur Behandlung von Patienten mit (Haut)transplantaten und hierbei insbesondere zur Behandlung von schlecht perfundierten Lappenplastiken eingesetzt. Die beiden vorab genannten hämodynamischen Größen, die arterielle Durchblutung sowie der venöse Rückfluss, stellen auch essenzielle Parameter des Therapieerfolgs chirurgischer Lappenplastiken dar. Als Lappenplastiken werden operative plastisch-chirurgische Techniken bezeichnet, die Haut und/oder Gewebe von einer (entbehrlichen) Stelle des gleichen Individuums an eine neue gewünschte Stelle bringen. In der Regel handelt es sich um reine Hautlappen, es kann aber jedes Gewebe mit oder ohne Haut sowohl gestielt (also mit seinen zugehörigen blutversorgenden Gefäßen und Nerven) als auch frei (d. h. mit Anschluss der Blutgefäße an die Blutversorgung der neuen Umgebung) verpflanzt werden. Die funktionelle Akzeptanz des verpflanzten Gewebes ist dabei ausschließlich von der arteriellen Blutversorgung sowie einem geregelten venösem Abfluss abhängig. Eine NO-bedingte arterielle Vasodilatation verbessert die Durchblutung und somit die notwendige Versorgung der Lappenplastik und durch die antithrombogene Wirkung des NO wird ein venöser Abfluss bzw. Rückfluss des Blutes gefördert und erleichtert. Von außen eingesetzte NO-Präparate können daher den Erfolg einer auf Lappenplastik basierten Therapieoption sichern bzw. fördern.

In einer weiteren Ausführungsform stellt die Erfindung auch ein kosmetisches Verfahren bereit, bei dem das durch das erfindungsgemäße Verfahren hergestellte NO auf die Haut des Menschen einwirkt.

## DEFINITIONEN

Unter dem Begriff der "Behandlung" ist jegliche Anwendung der Vorrichtung am Individuum zu verstehen, die dazu dient, die Erkrankung symptomatisch oder kausal zu lindern oder sogar gänzlich zu unterdrücken oder das Voranschreiten der Erkrankung aufzuhalten, zu verzögern oder hinauszuschieben.

Im Kontext der vorliegenden Erfindung wird unter der "Prävention" die Vermeidung des Auftretens von Erkrankungen, und insbesondere von vaskulären oder Stoffwechsel-Erkrankungen verstanden und damit die Verringerung ihrer Verbreitung und die Verminderung ihrer Auswirkungen auf Morbidität und Mortalität der Bevölkerung. Die zentrale Strategie ist, die Auslösefaktoren von Krankheiten zurückzudrängen oder ganz auszuschalten.

Die Prävention umfasst hierbei sowohl primordiale Prävention, die Primärprävention, die Sekundärprävention, die Tertiärprävention und auch die Quartärprävention.

Primärprävention setzt vor Eintreten der Krankheit ein und zielt darauf ab, ein Neuauftreten einer Erkrankung zu verhindern. Die Primärprävention richtet sich an Risikogruppen, Gesunde und Personen ohne Krankheitssymptome.

Von der Primärprävention kann noch die primordiale Prävention abgegrenzt werden, die noch früher einsetzt. Bei ihr geht es darum, bereits dem Auftreten von Risikofaktoren vorzubeugen.

Sekundärprävention setzt beim Frühstadium einer Krankheit an. Sie dient der Früherkennung von Krankheiten und der Eindämmung ihres Fortschreitens (Progredienz) oder der Chronifizierung der Erkrankung. Oft ohne eine für die Betroffenen wahrnehmbare Krankheitssymptomatik hat der pathogenetische Prozess hier bereits seinen Anfang genommen. Zielgruppe sind Personen, die zwar als Gesunde oder Symptomlose an der Präventionsmaßnahme teilnehmen,

durch die diagnostische Maßnahme aber zu Patienten werden.

Tertiärprävention findet nach einer Akutbehandlung oder der Manifestation einer Erkrankung statt. Mit ihr sollen Folgeschäden und Rückfälle verhindert werden. Sie richtet sich an Patienten mit chronischen Beeinträchtigungen und an Rehabilitanden. Ein Beispiel ist hier die Verhinderung von Rezidiven bei Tumorerkrankungen.

Weiterhin gibt es noch die Quartäre Prävention, die die Verhinderung unnötiger Medizin oder Verhinderung von Überdosierungen zum Ziel hat und das Prinzip des «primum non nocere» als einen Grundpfeiler aller Medizin berücksichtigt.

In den Patentansprüchen verwendete Begriffe wie "umfassen", "aufweisen", "beinhalten", "enthalten" und dergleichen schließen weitere Elemente oder Schritte nicht aus. Die Verwendung des unbestimmten Artikels schließt eine Mehrzahl nicht aus.

## BEISPIELE

### Beispiel 1. Einstufiges pH-induziertes NO-Herstellungsverfahren

#### 1.1 Material:

- Eco physics CLD 822: Quantifizierung von NO
- Reaktionskammer: Quarzglas, ca. 100x100x10mm (ca. 100ml Volumen)
- Pufferlösung: 150 mM Essigsäure, 150 mM NaOH in Aqua-dest
- Base: 1M NaOH
- Sodium L-ascorbate
- 1M NaNO2

#### 1.2 Versuchsführung

0,56 g Natrium-L-ascorbat wurden in 98,6 ml Pufferlösung gelöst, in die Reaktionskammer überführt und 1,4 ml NaNO2 (1M) zugegeben. Die Natriumnitritkonzentration betrug demnach 14 mM, die Ascorbatkonzentration 28,3 mM. Für die Endlösung wurde ein pH-Wert von 5,0 gemessen.

Über einen Zeitraum von 60 min wurde in Abständen von jeweils 2-3 Minuten eine 200 µl Probe entnommen und der NO Gehalt mit Hilfe des CLD Systems quantifiziert.

#### 1.3 Ergebnisse

Die Ergebnisse der NO-Messungen in Abhängigkeit von der Reaktionszeit sind in Figur 1 dargestellt. Es ist ein kontinuierlicher Anstieg der NO-Konzentration zu beobachten, wobei nach 60 Minuten ein Wert erreicht wird, der einer Konzentration von 1,11 mM in der Flüssigkeit entspricht.

### Beispiel 2. Zweistufiges pH-induziertes NO-Herstellungsverfahren

Ziel dieses Versuchs war das Erreichen einer therapeutisch relevanten Endkonzentration über einen längeren Zeitraum durch aktive Änderung des pH-Wertes.

#### 2.1 Material:

Es wurde das gleiche Material wie in Beispiel 1 verwendet

#### 2.2 Versuchsführung

Es wurde zunächst analog zu Versuch 1 0,56g Natrium-L-ascorbat in 98,6 ml Pufferlösung gelöst, diese in die Reaktionskammer überführt und 1,4 ml NaNO2 (1M) zugegeben. Die Natriumnitritkonzentration betrug demnach 14 mM, die Ascorbatkonzentration 28,3 mM. Für die Endlösung wurde ein pH-Wert von 5,0 gemessen.

Über einen Zeitraum von insg. 45 min wurde in Abständen von jeweils 2-3 Minuten eine 200 µl Probe entnommen und der NO Gehalt mit Hilfe des CLD Systems quantifiziert.

### 2.3 Ergebnisse

Es ist zunächst ein kontinuierlicher Anstieg der NO-Konzentration zu beobachten, wobei im Zeitraum zwischen 10 und 15 min schrittweise insg. 1,5 ml NaOH (1M) zur Reaktionskammer zugegeben wurden, was eine finale pH-Wert Änderung auf pH 5,6 zur Folge hatte. Die NO-Konzentration sinkt in Folge zunächst und pendelt sich ab t = 20 min bei einem Wert von 250 +/- 50 $\mu$M ein.

### FIGURENLEGENDEN

Die Erfindung wird nachfolgend anhand der Figuren näher erläutert ohne die Erfindung auf dieses zu beschränken. Es zeigen:

Fig. 1: die Generierung von NO mittels Natriumnitrit als NO-Donor in einem Acetat-Puffer bei pH 5.0 unter Anwesenheit von Ascorbat über einen Zeitraum von einer Stunde (siehe Beispiel 1).

Fig. 2: die Generierung von NO gemäß der Erfindung mittels Natriumnitrit als NO-Donor in einem Acetat-Puffer unter Anwesenheit von Ascorbat mit einer ersten Phase von t=0 bis t= 600 sec bei pH 5.0, einer pH-Erhöhung von t=600 bis t=900 auf einen pH-Wert von 5.6 und einer anschließenden Phase der NO-Generierung bei diesem pH-Wert von 5.6 (siehe Beispiel 2).

## Patentansprüche

1. Verfahren zur Herstellung von Stickstoffmonoxid (NO) umfassend die folgenden Schritte:

   (a) Bereitstellen eines Trägermediums umfassend mindestens einen pH-labilen NO-Donor;
   (b) Einstellen des pH-Werts des Trägermediums auf einen pH-Wert, der die Zersetzung des mindestens einen pH-labilen NO-Donors unter Bildung von NO induziert;
   (c) Aufrechterhalten eines die NO-Bildung induzierenden pH-Werts für eine Zeitdauer von 15 Sekunden bis 60 Minuten, die die Bildung von 0,01 bis 2 mM NO erlaubt;
   (d) Erhöhen des pH-Werts des Trägermediums um mindestens eine pH-Wertstufe;
   (e) Optionale Zugabe eines weiteren mindestens einen Antioxidans;

   wobei das Trägermedium in Schritt (a) zusätzlich mindestens ein Antioxidans enthält oder das mindestens eine Antioxidans in Schritt (b) hinzugegeben wird.

2. Verfahren gemäß Anspruch 1, **dadurch gekennzeichnet, dass** das Trägermedium ausgewählt ist aus der Gruppe enthaltend Schaum, Gel, Creme und Flüssigkeit und bevorzugt eine wässrige Flüssigkeit ist.

3. Verfahren gemäß Anspruch 1 oder 2, **dadurch gekennzeichnet, dass** der mindestens eine pH-labile NO-Donor ausgewählt ist aus der Gruppe enthaltend anorganisches Nitritsalz, Alkylnitrite wie Isopentylnitrit, Diazeniumdiolat-Derivate, trans[RuCl([15]aneN4)NO]$^{2+}$, 6-Nitrobenzo[a]pyrol, S-Nitroso-Glutathion, S-Nitroso-Thiol, S-Nitroso-N-Acetyl-D-Penicillamin (SNAP), Nitroanilin-Derivate, 2-Methyl-2-Nitrosopropan, Imidazoyl-Derivate, Nitratester, Hydroxylnitrosamin, Hydroxylamin, Hydroxyharnstoff, Natrium-Nitroprussid, und bevorzugt ein anorganisches Nitritsalz ist.

4. Verfahren gemäß Anspruch 1 bis 3, **dadurch gekennzeichnet, dass** der mindestens eine pH-labile NO-Donor ausgewählt ist aus der Gruppe $LiNO_2$, $NaNO_2$, $KNO_2$, $RbNO_2$, $CsNO_2$, $FrNO_2$, $Be(NO_2)_2$, $Mg(NO_2)_2$, $Ca(NO_2)_2$, $Sr(NO_2)_2$, $Ba(NO_2)_2$, oder $Ra(NO_2)_2$ und Kombinationen hiervon und bevorzugt $NaNO_2$ ist.

5. Verfahren gemäß einem der vorangehenden Ansprüche, **dadurch gekennzeichnet, dass** das mindestens eine Antioxidans aus Schritt (a) und/oder Schritt (e) ausgewählt ist aus der Gruppe enthaltend Ascorbat und Derivate hiervon, Tocopherol, -trienol, - monoenol und Derivate hiervon, Butylhydroxyanisol (BHA), Butylhydroxytoluol (BHT), Glutathion, Cystein, Thiomilchsäure, alpha-Liponsäure, p-Cumarsäure, Ferulasäure, Sinapinsäure, Kaffeesäure, Gallussäure, Procatechusäure, Syringasäure, Vanillinsäure, polyphenolische Verbindungen aus der Gruppe der Anthocyane, Flavonoide oder Phytoöstrogene, und bevorzugt ein Gemisch aus einem Ascorbinsäure-Derivat und einem Tocopherol-Derivat ist.

6. Verfahren gemäß einem der vorangehenden Ansprüche, **dadurch gekennzeichnet, dass** das mindestens eine Antioxidans aus Schritt (a) ein Gemisch aus Ascorbat und (RS)-6-Hydroxy-2,5,7,8-tetramethylchroman-2-carbonsäure (Trolox) oder ein Gemisch aus Ascorbylpalmitat oder Ascorbylstearat und alpha-Tocopherol ist.

7. Verfahren gemäß einem der vorangehenden Ansprüche, **dadurch gekennzeichnet, dass** das Trägermedium im Schritt (b) Nitrit, Ascorbat und Trolox in einem molaren Verhältnis von 1 : 2-20 : 4-100 enthält, wobei das molare Verhältnis: Nitrit < Ascorbat < Trolox ist und bevorzugt ein molares Verhältnis von 1 : 5 : 10 ist.

8. Verfahren gemäß einem der vorangehenden Ansprüche, **dadurch gekennzeichnet, dass** der im Schritt (b) eingestellte pH-Wert zwischen 0,0 und 6,9, bevorzugt zwischen 2,0 und 6,0, besonders bevorzugt zwischen 5,0 und 6,0 und insbesondere bei 5,5 liegt.

9. Verfahren gemäß einem der vorangehenden Ansprüche, **dadurch gekennzeichnet, dass** die pH-Werteinstellung im Schritt (b) durch Zugabe eine Säure oder durch photolytische Spaltung eines photolatenten Säurebildners erzeugt wird.

10. Verfahren gemäß einem der vorangehenden Ansprüche, **dadurch gekennzeichnet, dass** der Schritt (c) eine Zeitdauer von zwischen 1 und 30 Minuten und bevorzugt von zwischen 5 und 20 Minuten aufweist.

11. Verfahren gemäß einem der vorangehenden Ansprüche, **dadurch gekennzeichnet, dass** die physiologisch relevante Menge an NO einer Menge von zwischen 0,05 bis 1 mM und besonders bevorzugt zwischen 0,1 und 0,5 mM aufweist.

12. Verfahren gemäß einem der vorangehenden Ansprüche, **dadurch gekennzeichnet, dass** die pH-Werterhöhung im Schritt (d) eine oder mehrere der folgenden Eigenschaften aufweist:

    (i) eine Erhöhung des pH-Wertes auf pH7 oder mehr ist;
    (ii) eine Erhöhung des pH-Werts auf einen pH-Wert beinhaltet, der mit einer reduzierten NO-Generierung einhergeht, so dass die Menge an neugebildetem NO der Menge am im Trägermedium abnehmenden NO entspricht.

13. Verfahren gemäß einem der vorangehenden Ansprüche, **dadurch gekennzeichnet, dass** zur pH-Werterhöhung im Schritt (d) eine Base oder ein basischer Puffer verwendet wird, ausgewählt aus der Gruppe enthaltend NaOH, KOH, $NH_4OH$, $Ca(OH)_2$, $NH_4OH$, Natriumhydrogencarbonat, Phosphatpuffer, Barbital-Acetatpuffer, 4-(2-Hydroxyethyl)-1-piperazinethanesulfon-säure (HEPES)-Puffer, Tris(hydroxymethyl)-aminomethan (TRIS)-Puffer, 4-(2-Hydroxyethyl)-piperazin-1 -propansulfonsäure (HEPPS)-Puffer, Barbital-Acetat-Puffer, Essigsäure-Acetat-Puffer, Kohlensäure-Silicat-Puffer, 2-(N-Morpholino)ethansulfonsäure (MES)-Puffer, Kohlensäure-Bicarbonat-Puffer, Citronensäure-Puffer oder Citrat-Puffer.

14. Verfahren gemäß einen der vorangehenden Ansprüche, **dadurch gekennzeichnet, dass** die pH-Werterhöhung in Schritt (d) zu einem pH-Wert führt, der zwischen 7,0 und 12,0, bevorzugt zwischen 7,0 und 9,0, besonders bevorzugt zwischen 7,0 und 8,0 und insbesondere bei 7,5 liegt.

15. Verfahren gemäß einem der vorangehenden Ansprüche, **dadurch gekennzeichnet, dass** das in Schritt (e) hinzugegebene mindestens eine Antioxidans ausgewählt ist aus der Gruppe enthaltend Glutathion, Cystein, , N-Acetylcystein, Dimercaptobernsteinsäure, Dimercaptopropansulfonsäure. Ethanthiol (Ethylmercaptan), Dithiothreitol (DTT), Dithioerythritol (DTE), Captopril, Coenzym A, Penicillamin, 1-Propanthiol, 2-Propanthiol, Homocystein, Mesna, Methanthiol (Methylmercaptan) und Thiophenol.

16. Verfahren gemäß einem der vorangehenden Ansprüche, **dadurch gekennzeichnet, dass** nach Schritt (d) oder (e) das Trägermedium zur photolytischen Zersetzung des NO-Donors unter Bildung von NO mit Licht bestrahlt wird.

17. Kosmetisches Verfahren umfassend die Einwirkung von NO auf die Haut eines Menschen, **dadurch gekennzeichnet, dass** ein Verfahren gemäß Anspruch 1 bis 16 verwendet wird.

## Claims

1. Method for the production of nitrogen monoxide (NO) comprising the following steps:

   (a) Providing a carrier medium comprising at least one pH-labile NO-donor;
   (b) setting the pH value of the carrier medium to a pH value that induces the decomposition of the at least one pH-labile NO-donor in association with the production of NO;
   (c) maintaining a pH value that induces the NO-production for a period of time ranging from 15 seconds to 60 minutes that permits the production of 0.01 to 2 mM NO;
   (d) increasing the pH value of the carrier medium by at least one pH level;
   (e) as an option, adding at least one additional antioxidant;

   whereby the carrier medium in step (a) contains, in addition, at least one antioxidant or the at least one antioxidant is added in step (b).

2. Method according to claim 1, **characterised in that** the carrier medium is selected from the group containing foam, gel, cream, and liquid, and preferably is an aqueous liquid.

3. Method according to claim 1 or 2, **characterised in that** the at least one pH-labile NO-donor is selected from the group containing inorganic nitrite salt, alkyl nitrites such as isopentyl nitrite, diazeniumdiolate derivatives, trans[RuCl([15]aneN4)NO]$^{2+}$, 6-nitrobenzo[a]pyrol, S-nitroso-glutathion, S-nitrosothiol, S-nitroso-N-acetyl-D-penicillamine (SNAP), nitroaniline derivatives, 2-methyl-2-nitrosopropane, imidazoyl derivatives, nitrate esters, hydroxylnitrosamine, hydroxylamine, hydroxyurea, sodium nitroprusside, and preferably is an inorganic nitrite salt.

4. Method according to claims 1 to 3, **characterised in that** the at least one pH-labile NO-donor is selected from the group $LiNO_2$, $NaNO_2$, $KNO_2$, $RbNO_2$, $CsNO_2$, $FrNO_2$, $Be(NO_2)_2$, $Mg(NO_2)_2$, $Ca(NO_2)_2$, $Sr(NO_2)_2$, $Ba(NO_2)_2$ or $Ra(NO_2)_2$, and combinations thereof, and preferably is $NaNO_2$.

5. Method according to any one of the preceding claims, **characterised in that** the at least one antioxidant from step (a) and/or step (e) is selected from the group containing ascorbate and derivatives thereof, tocopherol, -trienol, -monoenol, and derivatives thereof, butylhydroxyanisol (BHA), butylhydroxytoluene (BHT), glutathione, cysteine, thiolactic acid, alpha-lipoic acid, p-cumaric acid, ferulaic acid, sinapinic acid, caffeic acid, gallic acid, protocatechuic acid, syringic acid, vanillic acid, polyphenolic compounds from the group of the anthocyans, flavonoids or phytooestrogens, and preferably is a mixture of an ascorbic acid derivative and a tocopherol derivative.

6. Method according to any one of the preceding claims, **characterised in that** the at least one oxidant from step (a) is a mixture of ascorbate and (RS)-6-hydroxy-2,5,7,8-tetramethylchroman-2-carboxylic acid (trolox) or a mixture of ascorbylpalmitate or ascorbylstearate and alpha-tocopherol.

7. Method according to any one of the preceding claims, **characterised in that** the carrier medium in step (b) contains nitrite, ascorbate, and trolox at a molar ratio of 1 : 2-20 : 4-100, whereby the molar ratio is nitrite < ascorbate < trolox and preferably is a molar ratio of 1 : 5 : 10.

8. Method according to any one of the preceding claims, **characterised in that** the pH value set in step (b) is between 0.0 and 6.9, preferably between 2.0 and 6.0, particularly preferably between 5.0 and 6.0, and in particular is 5.5.

9. Method according to any one of the preceding claims, **characterised in that** the pH value is set in step (b) by adding an acid or by photolytic cleavage of a photolatent acid producer.

10. Method according to any one of the preceding claims, **characterised in that** the step (b) takes a period of time of between 1 and 30 minutes and preferably of between 5 and 20 minutes.

11. Method according to any one of the preceding claims, **characterised in that** the physiologically relevant amount of NO comprises an amount of between 0.05 to 1 mM and particularly preferably between 0.1 and 0.5 mM.

12. Method according to any one of the preceding claims, **characterised in that** the increasing of the pH value in step (d) comprises one or more of the following features:

(i) is an increase of the pH value to pH 7 or more;
(ii) includes an increase of the pH value to a pH value that is associated with a reduced NO-production such that the amount of newly produced NO is equivalent to the decreasing amount of NO in the carrier medium.

**13.** Method according to any one of the preceding claims, **characterised in that** the pH is increased in step (d) by means of a base or an alkaline buffer selected from the group containing NaOH, KOH, $NH_4OH$, $Ca(OH)_2$, $NH_4OH$, sodium hydrogen carbonate, phosphate buffer, barbital-acetate buffer, 4-(2-hydroxyethyl)-1-piperazinethanesulfonic acid (HEPES) buffer, tris-(hydroxymethyl)-aminomethane (TRIS) buffer, 4-(2-hydroxyethyl)-piperazin-1-propanesulfonic acid (HEPPS) buffer, barbital-acetate buffer, acetic acid-acetate buffer, carbonic acid-silicate buffer, 2-(N-morpholino)ethanesulfonic acid (MES) buffer, carbonic acid-bicarbonate buffer, citric acid buffer or citrate buffer.

**14.** Method according to any one of the preceding claims, **characterised in that** the increasing of the pH value in step (d) leads to a pH that is between 7.0 and 12.0, preferably between 7.0 and 9.0, particularly preferably between 7.0 and 8.0, and in particular is 7.5.

**15.** Method according to any one of the preceding claims, **characterised in that** the at least one antioxidant added in step (e) is selected from the group containing glutathione, cysteine, N-acetylcysteine, dimercaptosuccinic acid, dimercaptopropanesulfonic acid, ethanethiol (ethylmercaptan), dithiothreitol (DTT), dithioerythritol (DTE), captopril, coenzyme A, penicillamine, 1-propanethiol, 2-propanethiol, homocysteine, mesna, methanethiol (methylmercaptan), and thiophenol.

**16.** Method according to any one of the preceding claims, **characterised in that,** after step (d) or (e), the carrier medium is irradiated with light for photolytic decomposition of the NO-donor in association with the production of NO.

**17.** Cosmetic method comprising the exposure of the skin of the human to NO, **characterised in that** a method according to claims 1 to 16 is used.

**Revendications**

**1.** Procédé de fabrication de monoxyde d’azote (NO) comprenant les étapes suivantes :

(a) mise à disposition d’un milieu de support comprenant au moins un donneur de NO au pH instable ;
(b) réglage de la valeur de pH du milieu de support sur une valeur de pH qui induit la décomposition de l’au moins un donneur de NO au pH instable en formant du NO;
(c) maintien d’une valeur de pH induisant la formation de NO pendant une durée de 15 secondes à 60 minutes qui permet la formation de 0,01 à 2 mM de NO;
(d) augmentation de la valeur de pH du milieu de support d’au moins un étage de valeur de pH ;
(e) addition optionnelle d’au moins un autre antioxydant ;

dans lequel le milieu de support à l’étape (a) contient en outre au moins un antioxydant ou l’au moins un antioxydant est ajouté à l’étape (b).

**2.** Procédé selon la revendication 1, **caractérisé en ce que** le milieu de support est sélectionné parmi le groupe contenant de la mousse, du gel, de la crème et du liquide, et est de préférence un liquide aqueux.

**3.** Procédé selon la revendication 1 ou 2, **caractérisé en ce que** l’au moins un donneur de NO au pH instable est sélectionné parmi le groupe contenant du sel de nitrite inorganique, des nitrites d’alkyle comme le nitrite d’isopentyle, des dérivés de diolate de diazénium, trans[RuCl([15]aneN4)NO]$^{2+}$, 6-nitrobenzo[a]pyrrole, S-nitroso-glutathion, S-nitroso-thiol, S-nitroso-N-acétyl-D-pénicillamine (SNAP), des dérivés de nitroaniline, 2-méthyl-2-nitrosopropane, des dérivés d’imidazoyle, des esters de nitrate, hydroxylnitrosamine, hydroxylamine, hydroxyurée, nitroprussiate de sodium et est de préférence un sel de nitrite inorganique.

**4.** Procédé selon les revendications 1 à 3, **caractérisé en ce que** l’au moins un donneur de NO au pH instable est sélectionné parmi le groupe $LiNO_2$, NaN02, KN02, RbN02, CsN02, FrN02, Be(N02)2, Mg(N02)2, Ca(N02)2, Sr(N02)2, Ba(N02)2 ou Ra(N02)2 et des combinaisons de ceux-ci, et est de préférence $NaNO_2$.

**5.** Procédé selon une des revendications précédentes, **caractérisé en ce que** l’au moins un antioxydant provenant

de l'étape (a) et/ou l'étape (e) est sélectionné parmi le groupe contenant l'ascorbate et des dérivés de celui-ci, le tocophérol, -triénol, -monoénol et des dérivés de ceux-ci, l'hydroxyanisole butylé (BHA), l'hydroxytoluène butylé (BHT), le glutathion, la cystéine, l'acide thiolactique, l'acide alpha-lipoïque, l'acide p-coumarique, l'acide férulique, l'acide sinapique, l'acide caféique, l'acide gallique, l'acide protocatéchique, l'acide syringique, l'acide vanillique, des composés polyphénoliques provenant du groupe des anthocyanes, flavonoïdes ou phytooestrogènes, et est de préférence un mélange d'un dérivé d'acide ascorbique et d'un dérivé de tocophérol.

6. Procédé selon une des revendications précédentes, **caractérisé en ce que** l'au moins un antioxydant provenant de l'étape (a) est un mélange d'ascorbate et d'acide (RS)-6-hydroxy-2,5,7,8-tétraméthylchromane-2-carboxylique (Trolox) ou un mélange de palmitate d'ascorbyle ou de stéarate d'ascorbyle et d'alpha-tocophérol.

7. Procédé selon une des revendications précédentes, **caractérisé en ce que** le milieu de support à l'étape (b) contient du nitrite, de l'ascorbate et du Trolox dans un rapport molaire de1/2 à 20/4 à 100, dans lequel le rapport molaire est : nitrite < ascorbate < Trolox et est de préférence un rapport molaire de 1/5/10.

8. Procédé selon une des revendications précédentes, **caractérisé en ce que** la valeur de pH réglée à l'étape (b) se situe entre 0,0 et 6,9, de préférence entre 2,0 et 6,0, de manière particulièrement préférée entre 5,0 et 6,0 et notamment à 5,5.

9. Procédé selon une des revendications précédentes, **caractérisé en ce que** le réglage de valeur de pH à l'étape (b) est généré par l'addition d'un acide ou par scission photolytique d'un formateur d'acide photolatent.

10. Procédé selon une des revendications précédentes, **caractérisé en ce que** l'étape (c) présente une durée comprise entre 1 et 30 minutes et de préférence comprise entre 5 et 20 minutes.

11. Procédé selon une des revendications précédentes, **caractérisé en ce que** la quantité de NO pertinente du point de vue physiologique présente une quantité comprise entre 0,05 et 1 mM et de manière particulièrement préférée entre 0,1 et 0,5 mM.

12. Procédé selon une des revendications précédentes, **caractérisé en ce que** l'augmentation de valeur de pH à l'étape (d) présente une ou plusieurs des propriétés suivantes :

(i) est une augmentation de la valeur de pH à pH7 ou plus ;
(ii) contient une augmentation de la valeur de pH à une valeur de pH qui s'accompagne d'une génération de NO réduite de sorte que la quantité de NO nouvellement formé correspond à la quantité de NO décroissant dans le milieu de support.

13. Procédé selon une des revendications précédentes, **caractérisé en ce qu'**une base ou un tampon basique est utilisé(e) pour l'augmentation de valeur de pH à l'étape (d), sélectionné(e) parmi le groupe contenant NaOH, KOH, $NH_4OH$, $Ca(OH)_2$, $NH_4OH$, hydrogénocarbonate de sodium, tampon phosphate, tampon d'acétate de barbital, tampon d'acide 4-(2-hydroxyéthyl)-1-pipérazineéthanesulfonique (HEPES), tampon de tris(hydroxyméthyl)aminométhane (TRIS), tampon d'acide 4-(2-hydroxyéthyl)-pipérazine-1-propanesulfonique (HEPPS), tampon d'acétate de barbital, tampon d'acétate d'acide acétique, tampon de silicate d'acide carboxylique, tampon d'acide 2-(N-morpholino)éthanesulfonique (MES), tampon de bicarbonate d'acide carboxylique, tampon d'acide citrique ou tampon de citrate.

14. Procédé selon une des revendications précédentes, **caractérisé en ce que** l'augmentation de valeur de pH à l'étape (d) conduit à une valeur de pH qui se situe entre 7,0 et 12,0, de préférence entre 7,0 et 9,0, de manière particulièrement préférée entre 7,0 et 8,0 et notamment à 7,5.

15. Procédé selon une des revendications précédentes, **caractérisé en ce que** l'au moins un antioxydant ajouté à l'étape (e) est sélectionné parmi le groupe contenant le glutathion, la cystéine, la N-acétylcystéine, l'acide dimercaptosuccinique, l'acide dimercaptopropanesulfonique, l'éthanethiol (éthylmercaptan), le dithiothréitol (DTT), le dithioérythritol (DTE), le captopril, la coenzyme A, la pénicillamine, le 1-propanethiol, le 2-propanethiol, l'homocystéine, le mesna, le méthanethiol (méthylmercaptan) et le thiophénol.

16. Procédé selon une des revendications précédentes, **caractérisé en ce que** le milieu de support est irradié de lumière après l'étape (d) ou (e) pour la décomposition photolytique du donneur de NO en formant du NO.

**17.** Procédé cosmétique comprenant l'effet de NO sur la peau d'un homme, **caractérisé en ce qu'**un procédé selon les revendications 1 à 16 est utilisé.

Fig. 1

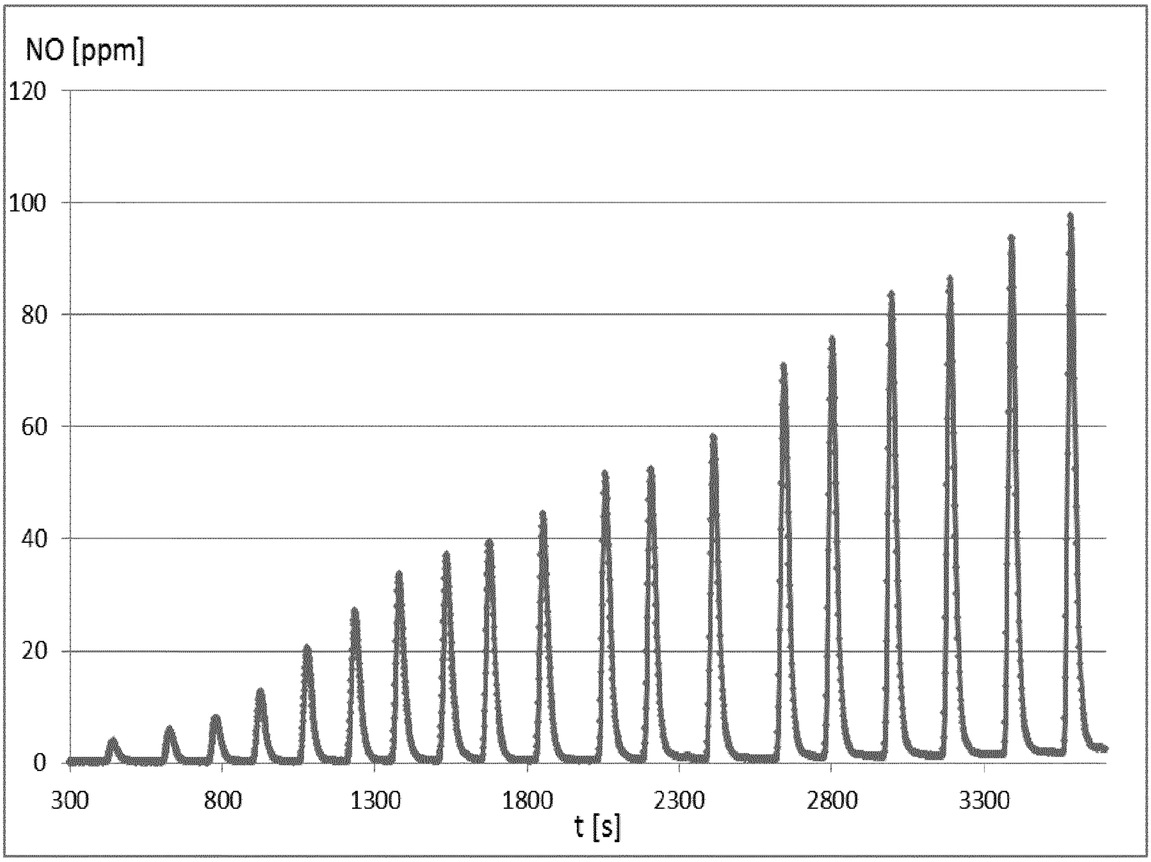

Fig. 2

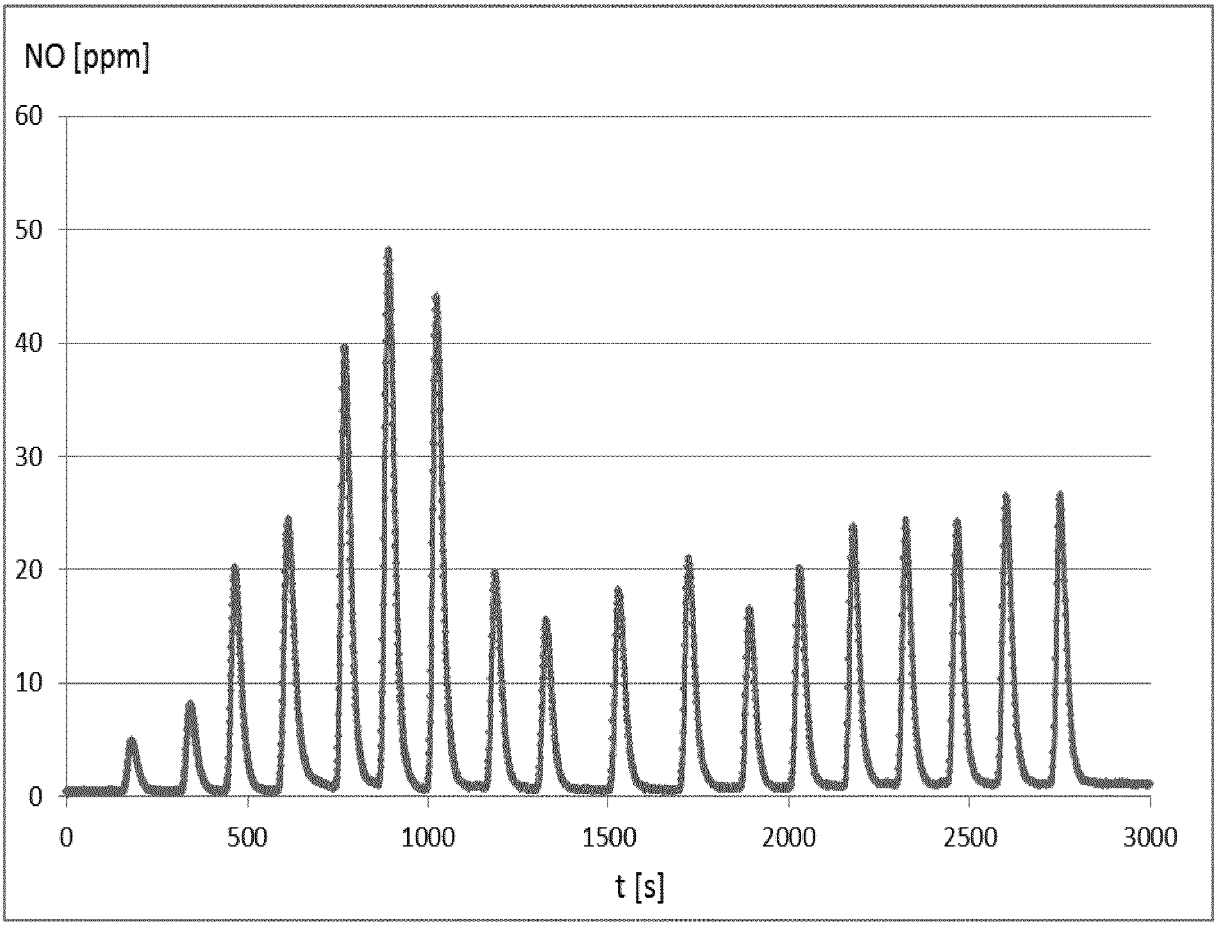

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 1903003 A1
- WO 2013063354 A
- US 20140335207 A1
- US 7105502 B
- US 7122529 B
- US 6673338 B
- US 20130224083 A1
- US 20030039697 A
- US 4151175 A
- US 3862333 A
- US 4694029 A
- EP 562897 A
- US 4399071 A
- WO 9846647 A
- US 5237059 A
- EP 571330 A
- EP 241423 A
- EP 139609 A
- EP 361907 A
- EP 199672 A
- EP 48615 A
- EP 12158 A
- EP 780729 A
- WO 20001097 A2
- GB 2348644 A
- WO 0026219 A
- WO 02101462 A1
- WO 2005097876 A1
- WO 2003050912 A
- EP 0898202 A1
- WO 9428075 A1
- WO 0192362 A1
- EP 0970085 A1
- WO 03033500 A1
- GB 2307473 A
- WO 9428075 A
- WO 9731033 A
- WO 199838195 A
- WO 200010964 A
- WO 199832756 A
- WO 199841524 A


### In der Beschreibung aufgeführte Nicht-Patentliteratur


- **KIRSCH et al.** *Biol. Chem,* 2002, vol. 383, 389-399
- Photoinitiators for Free Radical, Cationic & Anionic Photopolymerisation. **VON J.V. CRIVELLO ; K. DIETLIKER.** Chemistry & Technology of UV & EB Formulation for Coatings, Inks & Paints. J. Wiley and Sons, 1998, vol. III, 464-466
- **M. SHIRAI ; M. TSUNOOKA.** *Prog. Polym. Sci.,* 1996, vol. 21, 1-45